# FASCICULE DE BREVET EUROPEEN

(11) **EP 3 389 864 B1**
(45) Date de publication et mention de la délivrance du brevet: **26.05.2021**
(21) Numéro de dépôt: 16819982.6
(22) Date de dépôt: 07.12.2016
(51) Int. Cl.: B01J 38/00, B01J 23/26, B01J 27/12, B01J 23/92, B01J 38/02, B01J 38/44, B01J 38/46, C01B 9/08, C07C 17/20, B01J 37/26, B01J 27/132, B01J 37/16, B01J 37/12

(54) **FLUORATION CATALYTIQUE EN PHASE GAZEUSE AVEC DES CATALYSEURS A BASE DE CHROME.**
KATALYTISCHE GASPHASENFLUORIERUNG AUF CHROM-ENTHALTENDEN KATALYSATOR
CATALYTIC GAS PHASE FLUORINATION ON CHROMIUM TYPE CATALYST

(30) Priorité: 14.12.2015 FR 1562276
(43) Date de publication de la demande: 24.10.2018
(73) Titulaire: Arkema France, 92700 Colombes (FR)
(72) Inventeur: ANDRE, David, 69530 Brignais (FR); DEUR-BERT, Dominique, 69390 Charly (FR); GARRAIT, Dominique, 69390 Charly (FR); PIGAMO, Anne, 69340 Francheville (FR); WENDLINGER, Laurent, 69510 Soucieu En Jarrest (FR)
(74) Mandataire: Arkema Patent
(86) Numéro de dépôt international: PCT/FR2016/053236
(87) Numéro de publication internationale: WO 2017/103378

(56) Documents cités:
- EP-A1- 0 806 242
- WO-A1-2013/055722
- WO-A1-2013/055726
- WO-A1-2014/120493
- JP-A- H0 592 141
- US-A1- 2009 240 090
- US-A1- 2012 271 070

## Description

### Domaine de l'invention

La présente invention concerne un procédé de fluoration catalytique en phase gazeuse. De préférence, la présente invention concerne un procédé catalytique en phase gazeuse pour la fluoration d'un composé chloré en un composé fluoré en présence de fluorure d'hydrogène. En particulier, la présente invention concerne un procédé selon lequel la réaction de fluoration est réalisée en présence d'un catalyseur qui est régénéré.

### Arrière-plan de la présente invention

Le protocole de Montréal pour la protection de la couche d'ozone a exigé la suppression progressive de l'utilisation de chlorofluorocarbures (CFC). Des matériaux plus respectueux de la couche d'ozone, tels que les hydrofluorocarbures (HFC), p. ex. HFC-134a, ont remplacé les chlorofluorocarbures. Ces derniers composés se sont révélés être des gaz à effet de serre, provoquant un réchauffement planétaire. Ils ont été régulés par le protocole de Kyoto sur le changement climatique. Les préoccupations continuelles relatives au changement climatique de la planète entraînent un besoin croissant de développer des technologies pour remplacer celles qui présentent un potentiel d'appauvrissement de la couche d'ozone (ODP) élevé et un potentiel de réchauffement de la planète (GWP) élevé. Bien que les hydrofluorocarbures (HFC), n'étant pas des composés qui détruisent la couche d'ozone, aient été identifiés comme des alternatives aux chlorofluorocarbures (CFC) et aux hydrochlorofluorocarbures (HCFC) en tant que solvants, agents de nettoyage et fluides de transfert de chaleur, ils ont encore tendance à présenter un GWP significatif. Les hydrofluorooléfines (HFO) ont été identifiées comme des alternatives potentielles présentant un ODP nul et un faible GWP.

De nombreux documents décrivent par conséquent des procédés de fabrication de telles HFO, y compris HFO-1234yf.

Par exemple, WO 2007/079431 décrit des procédés de production d'oléfines fluorées, y compris d'hydrofluoropropènes. Les procédés qui sont décrits de manière générale sous la forme d'une seule réaction ou de deux réactions ou plus comprennent la fluoration d'un composé de formule C(X)ₘCCl(Y)ₙC(X)ₘ en au moins un composé de formule CF₃CF=CHZ, dans lesquelles X, Y et Z représentent chacun indépendamment H, F, Cl, I ou Br, et chaque m représente indépendamment 1, 2 ou 3, et n représente 0 ou 1. HFO-1234yf est préparé par fluoration d'HFCO-1233xf en 1,1,1,2-tétrafluoro-2-chloropropane (HCFC-244bb), suivie par une déshydrochloration. HFCO-1233xf est préparé par fluoration du précurseur chloré correspondant (CCl₂=CClCH₂Cl).

EP-A-939071 décrit, parmi de nombreuses possibilités, la fluoration en phase gazeuse d'un propène halogéné (selon une liste très longue) en un propène fluoré (y compris dans la liste HFO-1234yf).

WO 2008/054781 décrit divers procédés de production de divers fluoropropanes et halofluoropropènes par mise en réaction d'halopropanes ou d'halopropènes avec HF, éventuellement en présence d'un catalyseur. Il décrit un procédé de fabrication d'HFO-1234yf par mise en réaction de 2,3-dichloro-1,1,1-trifluoropropane (HCFC-243db) en présence d'HF, sur un catalyseur, notamment Cr/Co (98/2). Les produits de réaction comprennent HFO-1234yf et HFCO-1233xf, ce dernier étant le produit principal ; d'autres produits sont le 1-chloro-3,3,3-trifluoro-1-propène (HFCO-1233zd), ainsi que le 1,1,1,2,2-pentafluoropropane (HFC-245cb) et le 1,3,3,3-tétrafluoro-1-propène (HFO-1234ze).

WO 2008/002500 décrit un procédé de fabrication d'un mélange d'HFO-1234yf et d'HFO-1234ze par conversion catalytique de 1,1,1,2,3-pentafluoropropane (HFC-245eb) sur un catalyseur de déshydrofluoration.

WO 2008/040969 décrit un procédé comprenant la déshydrochloration d'HCFC-243db en HFCO-1233 (xf ainsi que zd), suivie par une réaction comprenant la formation de 1,1,1,2-tétrafluoro-2-chloropropane (HCFC-244bb) et la formation ultérieure de l'HFO-1234yf souhaité par déshydrochloration. L'Exemple 1 dudit document décrit une réaction en phase gazeuse à pression atmosphérique d'HCFC-243db avec HF sur un catalyseur de Zn/dioxyde de chrome, HFO-1234yf et HFCO-1233xf étant formés, ainsi qu'une petite quantité d'HFC-245cb.

WO 2009/015317 décrit la réaction d'un composé chloré qui peut être le 1,1,2,3-tétrachloro-1-propène (HCO-1230xa), le 1,1,1,2,3-pentachloropropane (HCC-240db) ou le 2,3,3,3-tétrachloro-1-propène (HCO-1230xf) avec HF, en phase gazeuse, sur un catalyseur et en présence d'au moins un stabilisateur. Ce procédé rend possible d'obtenir du 2-chloro-3,3,3-trifluoro-1-propène (HFCO-1233xf).

US 2009/0240090 décrit un procédé de fabrication de 2,3,3,3-tétrafluoro-1-propène (HFO-1234yf) partant d'un composé de formule (I) CX₂=CClCH₂X, ou de formule (II) CX₃CCl=CH₂ ou de formule (III) CX₃CHClCH₂X avec X=F, Cl, Br, I. Le procédé comprend trois étapes, qui peuvent être suivies par une purification. Le procédé comprend des étapes de recyclage permettant d'obtenir des conversions et des rendements plus élevés.

WO 2010/123154 concerne un procédé de production d'HFO-1234yf partant d'HFCO-1233xf, par mise en réaction de celui-ci avec HF en présence d'oxygène et d'un catalyseur comprenant de l'oxyde de chrome ou de l'oxyde de chrome fluoré.

WO2012/098421 et WO2012/098422 concernent des procédés pour la fluoration catalytique en phase gazeuse de 2-chloro-3,3,3-trifluoro-1-propène ou de 1,1,1,2,3-pentachloropropane pour produire du 2,2,2,3-tétrafluoropropène. La régénération du catalyseur est réalisée en présence d'un oxydant

JP H05 92141 A décrit un procédé de régénération d'un catalyseur utilisé dans un procédé de fluoration d'alcanes ou d'alcènes chlorés dans lequel l'agent réducteur est choisi parmi l'hydrogène, le monoxyde de carbone ou le monoxyde d'azote.

Il existe encore un besoin pour un procédé amélioré pour la fabrication de fluorooléfines telles qu'HFO-1234yf, ayant en particulier un taux de conversion amélioré et/ou une sélectivité améliorée et/ou étant efficace pendant une période de temps plus longue.

### Résumé de l'invention

Selon un premier aspect, la présente invention concerne un procédé de fluoration d'un composé chloré alcane ou alcène en C3 contenant au moins un atome de chlore en un composé fluoré alcane ou alcène en C3 contenant au moins un atome de fluor, comprenant les étapes suivantes :
(a) la mise en contact, dans un réacteur, du composé chloré avec du fluorure d'hydrogène en phase gazeuse en présence d'un catalyseur de fluoration pour produire le composé fluoré, le nombre d'atomes de fluor dans le composé fluoré étant supérieur au nombre d'atomes de fluor dans le composé chloré et
(b) la régénération du catalyseur de fluoration utilisé à l'étape a),
   l'étape (b) de régénération du catalyseur de fluoration comprenant (c) le traitement dudit catalyseur de fluoration avec un courant gazeux contenant un oxydant pour former un catalyseur de fluoration oxydé, et (d) le traitement du catalyseur de fluoration oxydé obtenu à l'étape (c) avec un mélange gazeux comprenant un agent réducteur et un gaz inerte ; le catalyseur régénéré à l'étape b) étant réutilisé à l'étape a) l'agent réducteur étant choisi dans le groupe constitué par les hydrohalocarbures en C₁-C₁₀; ledit catalyseur étant sélectionné parmi le groupe consistant en l'oxyfluorure de chrome, des oxydes de chrome, des halogénures de chrome et leurs mélanges.

Le présent procédé permet l'amélioration de la conversion ou de la sélectivité de la réaction. En effet, il a été observé qu'en soumettant le catalyseur de fluoration à une étape de régénération tel que nécessaire selon l'invention, la présence de sous-produits est limitée ou évitée, en particulier lorsque le catalyseur de fluoration ainsi régénéré est utilisé dans l'étape a).

Selon un autre mode de réalisation, le gaz inerte est choisi parmi l'azote, l'hélium, l'argon, leurs mélanges. De préférence, lorsque l'agent réducteur est un hydrohalocarbure en C₁-C₁₀, le mélange gazeux comprend de l'HF.

Selon un mode de réalisation préféré, l'agent réducteur peut être un agent réducteur gazeux.

Selon un mode de réalisation préféré, le mélange gazeux de l'étape (d) comprend de 1 à 10 % en volume d'agent réducteur, de préférence de 2 à 9 % en volume, de manière davantage préférée de 3 à 7 % en volume, par rapport au volume total du mélange gazeux.

De préférence, le mélange gazeux de l'étape (d) comprend de l'hydrogène et de l'azote ou de l'argon, de préférence consiste en l'hydrogène et l'azote ; ou le mélange gazeux de l'étape (d) comprend un hydrohalocarbures en C₂-C₆, de l'azote ou de l'argon, et de l'HF, de préférence le mélange gazeux de l'étape (d) comprend un hydrohalocarbure en C3, de l'azote et de l'HF.

De préférence, l'étape d) est réalisée à une température allant de 100 °C à 450 °C, avec une durée de contact de 1 à 100 s, de préférence de 1 à 75 s, de manière davantage préférée de 5 à 50 s, pendant une durée supérieure à 1 heure, de préférence de 1 à 50 heures, en particulier de 4 à 25 heures.

Selon un mode de réalisation préféré, le composé chloré peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb), le 1,1,1,3,3-pentachloropropane (HCC-240fa), le 1,1,3,3-tétrachloropropène (HCO-1230za), le 1,3,3,3-tétrachloropropène (HCO-1230zd), le 1-chloro-3,3,3-trifluoropropène (HCFO-1233zd), le 1,1,1,3-tétrachloropropane (HCC-250fb), le 1,1,3-trichloropropène (HCO-1240za), le 3,3,3-trichloropropène (HCO-1240zf) ; de manière davantage préférée, le composé chloré peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb).

Selon un mode de réalisation préféré, le composé fluoré est le 2,3,3,3-tétrafluoro-1-propène (HFO-1234yf), le 2-chloro-1,1,1,2-tétrafluoropropane (HCFC-244bb), le 1,1,1,2,2-pentafluoropropane (HFC-245cb), le 1,1,1,3,3-pentafluoropropane (HFC-245fa), le 1,1,1,3-tétrafluoro-3-chloropropane (HCFC-244fa), le 1-chloro-3,3,3-trifluoropropène (HCFO-1233zd), le 1,3,3,3-tétrafluoropropène (HFO-1234ze), le 3,3,3-trifluoropropène (HFO-1243zf) et le 2-chloro-3,3,3-trifluoro-1-propène (HFCO-1233xf).

De préférence, le composé chloré et le composé fluoré sont différents. Par exemple, lorsque le 2-chloro-3,3,3-trifluoro-1-propène (HFCO-1233xf) est le composé chloré, alors le composé fluoré obtenu à l'étape a) est différent du 2-chloro-3,3,3-trifluoro-1-propène (HFCO-1233xf). En outre, lorsque le 2-chloro-3,3,3-trifluoro-1-propène (HFCO-1233xf) est le composé fluoré obtenu à l'étape a), le composé chloré utilisé en tant que matériau de départ à l'étape a) est différent du 2-chloro-3,3,3-trifluoro-1-propène (HFCO-1233xf).

Selon un mode de réalisation préféré, la fluoration du composé chloré en un composé fluoré est :
- 2-chloro-3,3,3-trifluoro-1-propène (HFCO-1233xf) en 2,3,3,3-tétrafluoro-1-propène (HFO-1234yf) ;
- 1,1,1,2,3-pentachloropropane (HCC-240db) en 2,3,3,3-tétrafluoro-1-propène (HFO-1234yf) ;
- 1,1,2,2,3-pentachloropropane (HCC-240aa) en 2,3,3,3-tétrafluoro-1-propène (HFO-1234yf) ;
- 2,3-dichloro-1,1,1-trifluoropropane (HCFC-243db) en 2,3,3,3-tétrafluoro-1-propène (HFO-1234yf) ;
- 1,1,2,3-tétrachloro-1-propène (HCO-1230xa) en 2,3,3,3-tétrafluoro-1-propène (HFO-1234yf) ;
- 2,3,3,3-tétrachloro-1-propène (HCO-1230xf) en 2,3,3,3-tétrafluoro-1-propène (HFO-1234yf) ;
- 1,1,1,2,3-pentachloropropane (HCC-240db) en 2-chloro-3,3,3-trifluoro-1-propène (HFCO-1233xf) ;
- 1,1,2,2,3-pentachloropropane (HCC-240aa) en 2-chloro-3,3,3-trifluoro-1-propène (HFCO-1233xf) ;
- 2,3-dichloro-1,1,1-trifluoropropane (HCFC-243db) en 2-chloro-3,3,3-trifluoro-1-propène (HFCO-1233xf) ;
- 1,1,2,3-tétrachloro-1-propène (HCO-1230xa) en 2-chloro-3,3,3-trifluoro-1-propène (HFCO-1233xf) ;
- 2,3,3,3-tétrachloro-1-propène (HCO-1230xf) en 2-chloro-3,3,3-trifluoro-1-propène (HFCO-1233xf) ;
- 2-chloro-3,3,3-trifluoro-1-propène (HFCO-1233xf) en 2-chloro-1,1,1,2-tétrafluoropropane (HCFC-244bb) ;
- 1,1,1,2,3-pentachloropropane (HCC-240db) en 2-chloro-1,1,1,2-tétrafluoropropane (HCFC-244bb) ;
- 1,1,2,2,3-pentachloropropane (HCC-240aa) en 2-chloro-1,1,1,2-tétrafluoropropane (HCFC-244bb) ;
- 2,3-dichloro-1,1,1-trifluoropropane (HCFC-243db) en 2-chloro-1,1,1,2-tétrafluoropropane (HCFC-244bb) ;
- 1,1,2,3-tétrachloro-1-propène (HCO-1230xa) en 2-chloro-1,1,1,2-tétrafluoropropane (HCFC-244bb) ;
- 2,3,3,3-tétrachloro-1-propène (HCO-1230xf) en 2-chloro-1,1,1,2-tétrafluoropropane (HCFC-244bb) ;
- 1,1,1,2,3-pentachloropropane (HCC-240db) en 1,1,1,2,2-pentafluoropropane (HFC-245cb) ;
- 1,1,2,2,3- pentachloropropane (HCC-240aa) en 1,1,1,2,2-pentafluoropropane (HFC-245cb) ;
- 2,3-dichloro-1,1,1-trifluoropropane (HCFC-243db) en 1,1,1,2,2-pentafluoropropane (HFC-245cb) ;
- 1,1,2,3-tétrachloro-1-propène (HCO-1230xa) en 1,1,1,2,2-pentafluoropropane (HFC-245cb) ;
- 2,3,3,3-tétrachloro-1-propène (HCO-1230xf) en 1,1,1,2,2-pentafluoropropane (HFC-245cb).

Le catalyseur de fluoration est sélectionné dans le groupe consistant en l'oxyfluorure de chrome, des oxydes de chrome, des halogénures de chrome et leurs mélanges. Les halogénures de chrome désignent les fluorures de chrome et/ou les chlorures de chrome.

Selon un mode de réalisation préféré, le catalyseur de fluoration peut également contenir un ou plusieurs co-catalyseurs comprenant un sel d'un métal de transition choisi dans le groupe constitué par Co, Zn, Mn, Mg, V, Mo, Te, Nb, Sb, Ta, Ni ; ou un sel phosphoreux.

Selon un mode de réalisation préféré, le procédé comprend une étape d'activation réalisée avant l'étape (a) qui comprend une première étape de mise en contact du catalyseur de fluoration avec un courant gazeux contenant un oxydant sélectionné parmi l'oxygène, l'air, le chlore ou un mélange d'oxygène et d'azote.

Selon un mode de réalisation préféré, l'étape d'activation peut également comprendre une seconde étape, après la première étape, de traitement du catalyseur de fluoration obtenu après la première étape un mélange gazeux comprenant un agent réducteur et un gaz inerte, ledit agent réducteur étant l'hydrogène ou un hydrohalocarbure en C₂-C₆-

Selon un mode de réalisation préféré, les étapes (a) et (b) peuvent être réalisées en alternance.

Selon un mode de réalisation préféré, les étapes (a) et (b) sont réalisées dans un seul réacteur. Par ailleurs, l'étape d'activation telle que définie dans le présent document peut également être réalisée dans le même réacteur que celui utilisé pour les étapes (a) et (b) selon le présent procédé.

Selon un mode de réalisation préféré, une purge du réacteur peut être réalisée avant et/ou après l'étape (b), c.-à-d. entre la réaction de fluoration et l'étape de régénération et/ou entre la fin de l'étape de régénération et la réaction de fluoration en utilisant le catalyseur de fluoration ainsi régénéré. Ladite purge peut être réalisée en maintenant le réacteur sous vide ou en introduisant un courant d'azote dans le réacteur afin de remplacer les composants gazeux contenus dans le réacteur avant ou après l'étape b). En variante, la purge peut être réalisée en introduisant un courant d'un oxydant tel que l'air ou un mélange d'oxygène et d'azote dans le réacteur afin de remplacer les composants gazeux contenus dans le réacteur avant ou après l'étape b). La purge peut être réalisée à une température allant de la température ambiante à 400 °C ; de préférence à une pression absolue allant de la pression atmosphérique à 5 bar ; et de préférence pendant une durée allant de 1 heure à 50 heures.

Selon un second aspect, la présente invention concerne un procédé de fluoration d'un 2,3,3,3-tétrafluoropropène en 1,1,1,2,2-pentafluoropropane, comprenant les étapes suivantes :
(a) la mise en contact, dans un réacteur, de 2,3,3,3-tétrafluoropropène avec du fluorure d'hydrogène en phase gazeuse en présence d'un catalyseur de fluoration pour produire le 1,1,1,2,2-pentafluoropropane, et
(b) la régénération du catalyseur de fluoration utilisé à l'étape a),
l'étape (b) de régénération du catalyseur de fluoration comprenant (c) le traitement dudit catalyseur de fluoration avec un courant gazeux contenant un oxydant pour former un catalyseur de fluoration oxydé, et (d) le traitement du catalyseur de fluoration oxydé obtenu à l'étape (c) avec un mélange gazeux comprenant un agent réducteur ; le catalyseur régénéré à l'étape b) étant réutilisé à l'étape a) et l'agent réducteur étant choisi dans le groupe constitué par les hydrohalocarbures en C₁-C₁₀; ledit catalyseur étant sélectionné parmi le groupe consistant en l'oxyfluorure de chrome, des oxydes de chrome, des halogénures de chrome et leurs mélanges.

L'agent réducteur, l'oxydant, le catalyseur, l'étape de régénération et l'étape d'activation sont définis précédemment et/ou ultérieurement au regard du premier aspect de la présente invention.

### Description détaillée de la présente invention

Le terme « hydrohalocarbure en C₁-C₁₀ », tel qu'utilisé dans le présent document, désigne un alcane en C₁-C₁₀, un alcène en C₂-C₁₀, un alcyne en C₂-C₁₀ portant au moins un atome d'halogène, l'atome d'halogène étant de préférence F ou Cl. De préférence, hydrohalocarbure en C₁-C₁₀ désigne un alcane en C₂-C₆ ou un alcène en C₂-C₆ portant au moins un atome d'halogène, de préférence F ou Cl. De manière davantage préférée, l'hydrohalocarbure en C₁-C₁₀ peut être un composé chloré tel que défini ci-après, c.-à-d. un alcane en C₂-C₆ ou un alcène en C₂-C₆ portant au moins un atome de chlore. En particulier, l'hydrohalocarbure en C₁-C₁₀ est est un hydrohalocarbure en C₃ comportant au moins un atome de chlore.

La présente invention concerne un procédé de fluoration d'un composé. Il a été étonnamment découvert qu'en réalisant une régénération du catalyseur de fluoration utilisé dans le procédé de fluoration, la présence de sous-produits peut être limitée ou évitée.

Selon un premier aspect, la présente invention concerne par conséquent un procédé de fluoration d'un composé chloré en un composé fluoré. Ledit procédé comprend les étapes (a) de mise en contact du composé chloré avec du fluorure d'hydrogène en phase gazeuse en présence d'un catalyseur de fluoration pour produire le composé fluoré, le nombre d'atomes de fluor dans le composé fluoré étant supérieur au nombre d'atomes de fluor dans le composé chloré et (b) de régénération dudit catalyseur de fluoration utilisé à l'étape (a). En particulier, l'étape (b) de régénération du catalyseur de fluoration comprend (c) le traitement dudit catalyseur de fluoration avec un courant gazeux contenant un oxydant pour former un catalyseur de fluoration oxydé, et (d) le traitement du catalyseur de fluoration oxydé obtenu à l'étape (c) avec un mélange gazeux comprenant un agent réducteur.

Le « composé chloré » peut être toute molécule contenant un atome de chlore, et le « composé fluoré » peut être toute molécule contenant un atome de fluor.

De préférence, le composé chloré est un alcane en C₂-C₆ ou un alcène en C₂-C₆ linéaire ou ramifié portant au moins un atome de chlore. Le terme « alcane en C₂-C₆ » désigne un alcane qui contient 2, 3, 4, 5 ou 6 atomes de carbone. Le terme « alcène en C₂-C₆ » désigne un alcène qui contient 2, 3, 4, 5 ou 6 atomes de carbone. Le composé chloré peut être un alcane en C₂-C₅, de préférence un alcane en C₂-C₄, de manière davantage préférée un alcane en C₃-C₄, de manière préférée entre toutes un alcane en C₃, portant au moins un atome de chlore. Le composé chloré peut être un alcène en C₂-C₅, de préférence un alcène en C₂-C₄, de manière davantage préférée un alcène en C₃-C₄, de manière préférée entre toutes un alcène en C₃, portant au moins un atome de chlore. Ledit composé chloré peut porter au moins un atome de chlore, de préférence au moins deux atomes de chlore. Ledit composé chloré peut porter un, deux, trois, quatre, cinq ou six atomes de chlore. Par conséquent, ledit composé chloré peut être un alcane en C₂-C₆ ou un alcène en C₂-C₆ linéaire ou ramifié portant de un à six atomes de chlore, de préférence de un à cinq atomes de chlore ou de deux à cinq atomes de chlore. L'alcane en C₂-C₆ ou l'alcène en C₂-C₆ linéaire ou ramifié portant au moins un atome de chlore tel que défini dans le présent document peut également porter un ou plusieurs atomes d'halogène en plus du ou des atomes de chlore, ledit atome d'halogène étant choisi parmi F, I et Br ; de préférence F. Selon un mode de réalisation préféré, le nombre total d'atomes d'halogène dans le composé chloré tel que défini dans le présent document peut être de 2 à 5 atomes d'halogène, de préférence de 3 à 5 atomes d'halogène, de manière davantage préférée de 4 à 5 atomes d'halogène choisis parmi F, Cl, Br et I ; au moins un étant un atome de chlore.

De préférence, le composé chloré peut être un composé alcane en C₃ contenant au moins un atome de chlore, de préférence au moins deux atomes de chlore ; et de préférence de 0 à 5 atomes de fluor. Le composé chloré peut être un composé alcane en C₃ contenant un, deux, trois, quatre, cinq ou six atomes de chlore ; et de préférence aucun atome de fluor, un, deux, trois, quatre ou cinq atomes de fluor. De préférence, le composé chloré peut être un composé alcane en C₃ contenant quatre ou cinq atomes d'halogène choisis parmi Cl et F ; au moins un étant un atome de chlore, de préférence au moins deux étant un atome de chlore. De manière davantage préférée, le composé chloré peut être un composé alcane en C₃ contenant quatre ou cinq atomes d'halogène choisis parmi Cl et F, parmi lesquels au plus quatre atomes d'halogène sont des atomes de fluor, et au moins un est un atome de chlore, de préférence au moins deux sont un atome de chlore.

De préférence, le composé chloré peut être un composé alcène en C₃ contenant au moins un atome de chlore, de préférence au moins deux atomes de chlore ; et de préférence de 0 à 3 atomes de fluor. Le composé chloré peut être un composé alcène en C₃ contenant un, deux, trois ou quatre atomes de chlore ; et de préférence aucun atome de fluor, un, deux ou trois atomes de fluor. De préférence, le composé chloré peut être un composé alcène en C₃ contenant trois ou quatre atomes d'halogène choisis parmi Cl et F ; au moins un étant un atome de chlore, de préférence au moins deux étant un atome de chlore. De manière davantage préférée, le composé chloré peut être un composé alcène en C₃ contenant trois ou quatre atomes d'halogène choisis parmi Cl et F, parmi lesquels au plus trois atomes d'halogène sont des atomes de fluor, et au moins un est un atome de chlore.

De manière davantage préférée, le composé chloré peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb), le 1,1,1,3,3-pentachloropropane (HCC-240fa), le 1,1,3,3-tétrachloropropène (HCO-1230za), le 1,3,3,3-tétrachloropropène (HCO-1230zd), le 1-chloro-3,3,3-trifluoropropène (HCFO-1233zd), le 1,1,1,3-tétrachloropropane (HCC-250fb), le 1,1,3-trichloropropène (HCO-1240za), le 3,3,3-trichloropropène (HCO-1240zf).

En particulier, le composé chloré peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb).

De préférence, le composé fluoré est un alcane en C₂-C₆ ou un alcène en C₂-C₆ linéaire ou ramifié portant au moins un atome de fluor. Le composé fluoré peut être un alcane en C₂-C₅, de préférence un alcane en C₂-C₄, de manière davantage préférée un alcane en C₃-C₄, de manière préférée entre toutes un alcane en C₃ portant au moins un atome de fluor. Le composé fluoré peut être un alcène en C₂-C₅, de préférence un alcène en C₂-C₄, de manière davantage préférée un alcène en C₃-C₄, de manière préférée entre toutes un alcène en C₃ portant au moins un atome de fluor.

Ledit composé fluoré peut être un alcane en C₂-C₆ ou un alcène en C₂-C₆ linéaire ou ramifié portant au moins un atome de fluor, de préférence au moins deux atomes de fluor, de manière davantage préférée au moins trois atomes de fluor. Le composé fluoré peut porter un, deux, trois, quatre ou cinq atomes de fluor. Le composé fluoré peut porter de un à cinq atomes de fluor, de préférence de deux à cinq atomes de fluor ou de trois à cinq atomes de fluor. L'alcane en C₂-C₆ ou l'alcène en C₂-C₆ linéaire ou ramifié portant au moins un atome de fluor tel que défini dans le présent document peut également porter un ou plusieurs atomes d'halogène en plus du ou des atomes de fluor, ledit atome d'halogène étant choisi parmi Cl, I et Br; de préférence Cl. Selon un mode de réalisation préféré, le nombre total d'atomes d'halogène dans le composé fluoré tel que défini dans le présent document peut être de 2 à 5 atomes d'halogène, de préférence de 3 à 5 atomes d'halogène, de manière davantage préférée de 4 ou 5 atomes d'halogène choisis parmi F, Cl, Br et I ; au moins un étant un atome de fluor, de préférence au moins deux étant un atome de fluor, de manière davantage préférée au moins trois étant un atome de fluor.

De préférence, le composé fluoré peut être un composé alcane en C₃ contenant au moins un atome de fluor, de préférence au moins deux atomes de fluor, de manière davantage préférée au moins trois atomes de fluor. Le composé fluoré peut être un alcane en C₃ contenant un, deux, trois, quatre ou cinq atomes de fluor ; et de préférence de 0 à 5 atomes de chlore, de manière davantage préférée de 0 à 4 atomes de chlore, en particulier de 0 à 3 atomes de chlore. De préférence, le composé fluoré peut être un composé alcane en C₃ contenant quatre ou cinq atomes d'halogène choisis parmi Cl et F ; au moins un étant un atome de fluor. De manière davantage préférée, le composé fluoré peut être un composé alcane en C₃ contenant quatre ou cinq atomes d'halogène choisis parmi Cl et F, parmi lesquels au plus trois, de préférence au plus deux, atomes d'halogène sont des atomes de chlore ; et au moins un est un atome de fluor, de préférence au moins deux sont un atome de fluor, de manière davantage préférée au moins trois sont un atome de fluor.

De préférence, le composé fluoré peut être un composé alcène en C₃ contenant au moins un atome de fluor, de préférence au moins deux atomes de fluor, de manière davantage préférée au moins trois atomes de fluor. Le composé fluoré peut être un composé alcène en C₃ contenant un, deux, trois ou quatre atomes de fluor ; et de préférence de 0 à 2 atomes de chlore, de manière davantage préférée de 0 à 1 atome de chlore. De préférence, le composé fluoré peut être un composé alcène en C₃ contenant quatre atomes d'halogène choisis parmi Cl et F ; au moins un étant un atome de fluor. De manière davantage préférée, le composé fluoré peut être un composé alcène en C₃ contenant quatre atomes d'halogène choisis parmi Cl et F, parmi lesquels au plus deux, de préférence au plus un, atomes d'halogène sont des atomes de chlore ; et au moins un est un atome de fluor, de préférence au moins deux sont un atome de fluor, de manière davantage préférée au moins trois sont un atome de fluor. En particulier, le composé fluoré peut être un alcène en C₃ contenant quatre atomes d'halogène, ces derniers étant des atomes de fluor. Le composé fluoré peut également être un composé alcène en C₃ contenant quatre atomes d'halogène, trois sur quatre étant des atomes de fluor, les atomes d'halogène restants étant des atomes de chlore.

De manière davantage préférée, le composé fluoré peut être choisi dans le groupe constitué par le 2,3,3,3-tétrafluoro-1-propène (HFO-1234yf), le 2-chloro-1,1,1,2-tétrafluoropropane (HCFC-244bb), le 1,1,1,2,2-pentafluoropropane (HFC-245cb), le 1,1,1,3,3-pentafluoropropane (HFC-245fa), le 1,1,1,3-tétrafluoro-3-chloropropane (HCFC-244fa), le 1-chloro-3,3,3-trifluoropropène (HCFO-1233zd), le 1,3,3,3-tétrafluoropropène (HFO-1234ze-E), le 3,3,3-trifluoropropène (HFO-1243zf) et le 2-chloro-3,3,3-trifluoro-1-propène (HFCO-1233xf).

Selon le présent procédé, la fluoration du composé chloré repose sur l'augmentation du degré de fluoration, c.-à-d. que le nombre d'atomes de fluor dans le composé fluoré est supérieur au nombre d'atomes de fluor dans le composé chloré. De préférence, pendant la réaction, au moins un substituant Cl dans le composé chloré est remplacé par un substituant F. De préférence, le composé chloré choisi pour la réaction et le composé fluoré obtenu à partir de celui-ci par la réaction de fluoration ont le même nombre d'atomes de carbone.

Selon un mode de réalisation préféré, la fluoration du composé chloré en un composé fluoré est :
- 2-chloro-3,3,3-trifluoro-1-propène (HFCO-1233xf) en 2,3,3,3-tétrafluoro-1-propène (HFO-1234yf) ;
- 1,1,1,2,3-pentachloropropane (HCC-240db) en 2,3,3,3-tétrafluoro-1-propène (HFO-1234yf) ;
- 1,1,2,2,3-pentachloropropane (HCC-240aa) en 2,3,3,3-tétrafluoro-1-propène (HFO-1234yf) ;
- 2,3-dichloro-1,1,1-trifluoropropane (HCFC-243db) en 2,3,3,3-tétrafluoro-1-propène (HFO-1234yf) ;
- 1,1,2,3-tétrachloro-1-propène (HCO-1230xa) en 2,3,3,3-tétrafluoro-1-propène (HFO-1234yf) ;
- 2,3,3,3-tétrachloro-1-propène (HCO-1230xf) en 2,3,3,3-tétrafluoro-1-propène (HFO-1234yf) ;
- 1,1,1,2,3-pentachloropropane (HCC-240db) en 2-chloro-3,3,3-trifluoro-1-propène (HFCO-1233xf) ;
- 1,1,2,2,3-pentachloropropane (HCC-240aa) en 2-chloro-3,3,3-trifluoro-1-propène (HFCO-1233xf) ;
- 2,3-dichloro-1,1,1-trifluoropropane (HCFC-243db) en 2-chloro-3,3,3-trifluoro-1-propène (HFCO-1233xf) ;
- 1,1,2,3-tétrachloro-1-propène (HCO-1230xa) en 2-chloro-3,3,3-trifluoro-1-propène (HFCO-1233xf) ;
- 2,3,3,3-tétrachloro-1-propène (HCO-1230xf) en 2-chloro-3,3,3-trifluoro-1-propène (HFCO-1233xf) ;
- 2-chloro-3,3,3-trifluoro-1-propène (HFCO-1233xf) en 2-chloro-1,1,1,2-tétrafluoropropane (HCFC-244bb) ;
- 1,1,1,2,3-pentachloropropane (HCC-240db) en 2-chloro-1,1,1,2-tétrafluoropropane (HCFC-244bb) ;
- 1,1,2,2,3-pentachloropropane (HCC-240aa) en 2-chloro-1,1,1,2-tétrafluoropropane (HCFC-244bb) ;
- 2,3-dichloro-1,1,1-trifluoropropane (HCFC-243db) en 2-chloro-1,1,1,2-tétrafluoropropane (HCFC-244bb) ;
- 1,1,2,3-tétrachloro-1-propène (HCO-1230xa) en 2-chloro-1,1,1,2-tétrafluoropropane (HCFC-244bb) ;
- 2,3,3,3-tétrachloro-1-propène (HCO-1230xf) en 2-chloro-1,1,1,2-tétrafluoropropane (HCFC-244bb) ;
- 1,1,1,2,3-pentachloropropane (HCC-240db) en 1,1,1,2,2-pentafluoropropane (HFC-245cb) ;
- 1,1,2,2,3-pentachloropropane (HCC-240aa) en 1,1,1,2,2-pentafluoropropane (HFC-245cb) ;
- 2,3-dichloro-1,1,1-trifluoropropane (HCFC-243db) en 1,1,1,2,2-pentafluoropropane (HFC-245cb) ;
- 1,1,2,3-tétrachloro-1-propène (HCO-1230xa) en 1,1,1,2,2-pentafluoropropane (HFC-245cb) ;
- 2,3,3,3-tétrachloro-1-propène (HCO-1230xf) en 1,1,1,2,2-pentafluoropropane (HFC-245cb).

La conversion du composé chloré en le composé fluoré comprend une conversion directe (c.-à-d. en une étape de réaction individuelle ou selon essentiellement un ensemble de conditions de réaction) et une conversion indirecte (c.-à-d. par deux étapes de réaction ou plus ou en utilisant plus d'un ensemble individuel de conditions de réaction).

La réaction de fluoration peut être réalisée avec :
- un rapport molaire HF généralement de 3:1 à 150:1, de préférence de 4:1 à 125:1, de manière davantage préférée de 5:1 à 100:1 ;
- une durée de contact de 3 à 100 s, de préférence de 4 à 75 s, de manière davantage préférée de 5 à 50 s ; et
- une pression allant de la pression atmosphérique à 20 bar, de préférence de 2 à 18 bar, de manière davantage préférée de 3 à 15 bar.

L'étape a) peut être réalisée à une température de 200 à 450 °C, de préférence de 250 °C à 400 °C, de manière davantage préférée de 280 °C à 380 °C. De préférence, la température de l'étape a) est la température du lit catalytique.

Afin d'empêcher une désactivation rapide du catalyseur pendant la réaction de fluoration, un oxydant (tel que l'oxygène ou le chlore) peut être ajouté, par exemple à un taux de 0,05 à 20 % en moles, de préférence de 0,1 à 15 % en moles, de manière davantage préférée de 0,5 à 10 % en moles, en particulier de 1 à 8 % en moles, par rapport au mélange de l'oxydant plus le composé chloré.

Selon un mode de réalisation préféré, une purge du réacteur peut être réalisée avant la régénération du catalyseur de fluoration selon l'étape (b) de la présente invention. La purge peut être réalisée en maintenant le réacteur sous vide ou en introduisant un courant d'azote dans le réacteur après la réalisation de l'étape (a) du présent procédé.

Selon un mode de réalisation préféré, les étapes a) et b) peuvent être réalisées en alternance. Lorsque l'étape a) alterne avec l'étape b), la durée de chaque étape peut être de 50 à 2 000 heures, avantageusement de 50 à 1500 heures, de préférence de 200 à 1 000 heures, et la durée de chaque étape de régénération peut être de 10 à 200 heures, de préférence de 15 à 60 heures.

### Catalyseur

Il peut s'agir par exemple d'un catalyseur à base d'un métal comprenant un oxyde de métal de transition ou un dérivé ou halogénure ou oxyhalogénure d'un tel métal. Des catalyseurs qui peuvent être utilisés sont qui sont utilisés sont sélectionnés dans le groupe consistant en l'oxyfluorure de chrome, les oxydes de chrome, les halogénures de chrome, le fluorure et oxyfluorure d'aluminium, un catalyseur supporté ou non supporté. On peut également se référer aux divulgations de WO-A-2007/079431, à la page 7, lignes 1 à 5 et 28 à 32, EP-A-939071, paragraphe [0022], WO 2008/054781 à la page 9 ligne 22 à la page 10 ligne 34, WO 2008/040969 dans la revendication 1.

Selon un mode de réalisation préféré, le catalyseur de fluoration peut comprendre un oxyfluorure de chrome, des oxydes de chrome, des halogénures de chrome et leurs mélanges. Le catalyseur de fluoration utilisé dans la présente invention peut être supporté ou non supporté. Selon un mode de réalisation préféré, le catalyseur d'oxyfluorure de chrome peut avoir une teneur en fluor de plus de 30 % en poids par rapport au poids total du catalyseur d'oxyfluorure de chrome, de préférence de 30 à 45% en poids. En variante, le catalyseur d'oxyfluorure de chrome peut avoir une teneur en fluor de moins de 30 % en poids par rapport au poids total du catalyseur d'oxyfluorure de chrome.

Le catalyseur de fluoration peut être de l'oxyfluorure de chrome, des oxydes de chrome, des halogénures de chrome supportés ou non supportés et leurs mélanges.

Selon un mode de réalisation préféré, le catalyseur est un catalyseur mixte supporté contenant à la fois du chrome et du nickel. Le rapport molaire Cr:Ni, par rapport à l'élément métallique, est généralement compris entre 0,5 et 5, par exemple entre 0,7 et 2, y compris proche de 1. Le catalyseur peut contenir de 0,5 à 20 % en poids de nickel.

En ce qui concerne les catalyseurs supportés, le support du catalyseur peut être choisi parmi les matériaux connus dans le domaine pour être compatibles avec HF à une température et une pression plus élevée. Par exemple, l'alumine fluorée, le charbon actif pré-fluoré, le graphite ou le graphite fluoré sont des supports de catalyseur appropriés. Le support est de préférence en aluminium. Il existe plusieurs supports possibles tels que l'alumine, l'alumine activée ou les dérivés d'aluminium. Ces dérivés comprennent les halogénures d'aluminium et les oxyhalogénures d'aluminium, par exemple décrits dans US 4,902,838, ou obtenus par le procédé d'activation. On peut se référer à WO 2009/118628, et notamment à la divulgation du catalyseur de la page 4, ligne 30 à la page 7, ligne 16.

Selon un autre mode de réalisation, le procédé utilise un catalyseur à base de Cr de grande aire de surface qui est de préférence non supporté. Un catalyseur préféré est un catalyseur d'oxyde de chrome non supporté de grande aire de surface.

L'un quelconque des catalyseurs définis dans le présent document peut avoir une aire de surface d'au moins 50 m²/g, de préférence de 50 à 300 m²/g, de manière davantage préférée de 70 à 250 m²/g, en particulier de 100 à 200 m²/g.

D'autres catalyseurs possibles sont les catalyseurs de fluoration à base de dioxyde de chrome comprenant du zinc ou de l'oxyde de zinc. La quantité totale du zinc ou d'un composé de zinc présente dans les catalyseurs de zinc/dioxyde de chrome peut être d'environ 0,01 % à environ 25 %, de préférence de 0,1 % à 25 %, commodément de 0,01 % à 6 % de zinc, et selon certains modes de réalisation de préférence 0,5 % en poids à 25 % en poids du catalyseur, de préférence d'environ 1 à 10 % en poids du catalyseur, de manière davantage préférée d'environ 2 à 8 % en poids du catalyseur, par exemple d'environ 4 à 6 % en poids du catalyseur. Selon d'autres modes de réalisation, le catalyseur comprend commodément 0,01 % à 1 %, de manière davantage préférée 0,05 % à 0,5 % de zinc. Les catalyseurs de zinc/dioxyde de chrome peuvent comprendre un métal supplémentaire ou un composé de celui-ci. Généralement, le métal supplémentaire est un métal bivalent ou trivalent, de préférence choisi parmi le nickel, le magnésium, l'aluminium et leurs mélanges. Généralement, le métal supplémentaire est présent en une quantité de 0,01 % en poids à environ 25 % en poids du catalyseur, de préférence d'environ 0,01 à 10 % en poids du catalyseur. D'autres modes de réalisation peuvent comprendre au moins environ 0,5 % en poids ou au moins environ 1 % en poids de métal supplémentaire. D'autres catalyseurs sont des catalyseurs de fluoration à base de dioxyde de chrome constitués de dioxyde de chrome amorphe ; d'oxyde de zinc en une quantité totale de zinc de 0,5 à 25 % en poids du catalyseur ; et d'oxyde de chrome cristallin en une quantité totale de 0,1 à 2,5 % en poids du catalyseur; le catalyseur étant supporté ou non supporté, tel que décrit dans EP 1 877 181.

Selon un mode de réalisation préféré, le catalyseur peut contenir, de préférence à un faible niveau, un ou plusieurs co-catalyseurs tels qu'un sel de Co, Zn, Mn, Mg, V, Mo, Te, Nb, Sb, Ta, P et Ni. Un co-catalyseur préféré peut être le nickel, le magnésium ou le zinc. Le catalyseur de chrome non supporté préféré peut éventuellement contenir de faibles niveaux d'un ou de plusieurs co-catalyseurs choisis parmi le cobalt, le nickel, le zinc, le manganèse, le magnésium ou un mélange de manganèse et de magnésium, préparé par des procédés connus dans le domaine, tel que par imprégnation, poudre mélangée et analogues.

La quantité de co-catalyseur, lorsqu'il est présent, peut varier de 1 à 20 % en poids, de préférence de 1 à 10 % en poids, de manière davantage préférée de 1 à 5 % en poids. Le co-catalyseur peut être ajouté au catalyseur par des procédés connus dans le domaine, tels que l'adsorption à partir d'une solution aqueuse ou organique, suivie par une évaporation du solvant. Le catalyseur préféré selon ce mode de réalisation est de l'oxyde de chrome pur avec du nickel ou du zinc en tant que co-catalyseur. En variante, le co-catalyseur peut être mélangé physiquement avec le catalyseur par broyage pour produire un mélange intime.

Avant l'activation, le catalyseur peut être soumis à une étape de séchage. Ladite étape de séchage peut comprendre le passage d'un gaz de séchage, de préférence de l'azote, sur le catalyseur. L'étape de séchage peut être réalisée à une pression allant de la pression atmosphérique jusqu'à 20 bar. La température du catalyseur pendant l'étape de séchage peut aller de la température ambiante jusqu'à 400 °C, de préférence d'environ 100 °C à environ 300 °C, pendant une durée de contact d'environ 1 à 100 s, de préférence d'environ 10 à 40 s, pendant approximativement 1 à 50 heures, de préférence entre 5 à 20 heures.

Après l'étape de séchage, le catalyseur peut être activé pour obtenir un meilleur niveau d'activité du catalyseur.

### Activation du catalyseur

Les présents inventeurs ont découvert que l'activation des catalyseurs précédents en utilisant un courant gazeux contenant un oxydant peut améliorer l'efficacité du procédé de fluoration.

Le procédé d'activation comprend l'activation du catalyseur en utilisant un agent d'activation ou deux agents d'activation, en deux étapes ou en une seule étape. Un des agents d'activation est un oxydant, tel que l'oxygène ou un mélange oxygène/azote ou l'air ou le chlore. L'autre agent d'activation peut être un mélange gazeux comprenant un agent réducteur.

Selon un premier mode de réalisation, le procédé d'activation comprend une étape de mise en contact du catalyseur de fluoration avec un courant gazeux contenant un oxydant. Le catalyseur de fluoration est traité avec l'oxydant. L'oxydant peut être un agent contenant de l'oxygène, de préférence choisi parmi l'air, l'oxygène, le chlore ou un mélange d'oxygène et d'azote. La température pendant le traitement avec l'oxydant peut aller de 250 à 500 °C, de préférence de 300 à 450 °C, de manière davantage préférée de 350 à 400 °C ; de préférence avec une durée de contact d'environ 1 à environ 200 s, de préférence de 1 à 150 s, de manière davantage préférée de 5 à 100 s ; et de préférence pendant une durée d'au moins 1 heure, de préférence d'au moins 2 heures, de manière davantage préférée d'au moins 4 heures, de manière préférée entre toutes d'au moins 10 heures, en particulier d'au moins 15 heures. Par conséquent, le traitement avec l'oxydant peut être réalisé pendant une durée de 1 à environ 1 500 heures, de préférence de 2 à 1 000 heures, de manière davantage préférée de 4 à 500 heures, de manière préférée entre toutes de 10 à 200 heures, en particulier de 15 à 150 heures.

Selon un autre mode de réalisation, l'étape d'activation comprend une première étape (i) de mise en contact du catalyseur de fluoration avec un courant gazeux contenant un oxydant, tel que défini précédemment, et une seconde étape (ii) de traitement du catalyseur de fluoration obtenu après l'étape (i) avec un mélange gazeux comprenant un agent réducteur.

Le mélange gazeux comprenant l'agent réducteur peut également comprendre un gaz inerte. Le gaz inerte peut être l'azote, l'hélium, l'argon, HF ou leurs mélanges. Alternativement, le gaz inerte peut comprendre l'azote, l'hélium, l'argon ou leurs mélanges. Le mélange gazeux peut comprendre de 1 à 10 % en volume d'agent réducteur, de préférence de 2 à 9 % en volume, de manière davantage préférée de 3 à 7 % en volume, par rapport au volume total du mélange gazeux.

L'agent réducteur peut être choisi dans le groupe constitué par l'hydrogène, le monoxyde de carbone, le monoxyde d'azote, le formaldéhyde, les alcanes en C₁-C₆ et les hydrohalocarbures en C₁-C₁₀. De préférence, l'agent réducteur peut être l'hydrogène, le formaldéhyde, un alcane en C₁-C₆ ou un hydrohalocarbure en C₁-C₁₀. En particulier, l'agent réducteur peut être un hydrohalocarbure en C₁-C₁₀, de préférence un composé chloré tel que défini précédemment. Lorsque l'agent réducteur est un composé chloré, il peut être identique ou différent du composé chloré utilisé à l'étape a) du présent procédé, de préférence identique à celui utilisé à l'étape a) du présent procédé. Lorsque l'agent réducteur est un hydrohalocarbure en C₁-C₁₀, de préférence un composé chloré, le mélange gazeux comprend un gaz inerte tel que l'azote, l'hélium, l'argon, HF ou leurs mélanges. De préférence, lorsque l'agent réducteur est un hydrohalocarbure en C₁-C₁₀, en particulier un hydrohalocarbure en C₃, le mélange gazeux comprend de l'HF.

Par conséquent, l'agent réducteur peut être un composé alcane en C₃ contenant au moins un atome de chlore, de préférence au moins deux atomes de chlore ; et de préférence de 0 à 5 atomes de fluor. L'agent réducteur peut être un composé alcane en C₃ contenant un, deux, trois, quatre, cinq ou six atomes de chlore ; et de préférence aucun atome de fluor, un, deux, trois, quatre ou cinq atomes de fluor. De préférence, l'agent réducteur peut être un composé alcane en C₃ contenant quatre ou cinq atomes d'halogène choisis parmi Cl et F ; au moins un étant un atome de chlore, de préférence au moins deux étant un atome de chlore. De manière davantage préférée, l'agent réducteur peut être un composé alcane en C₃ contenant quatre ou cinq atomes d'halogène choisis parmi Cl et F ; parmi lesquels au plus quatre atomes d'halogène sont des atomes de fluor, et au moins un est un atome de chlore, de préférence au moins deux sont un atome de chlore.

De préférence, l'agent réducteur peut être un composé alcène en C₃ contenant au moins un atome de chlore, de préférence au moins deux atomes de chlore ; et de préférence de 0 à 3 atomes de fluor. L'agent réducteur peut être un composé alcène en C₃ contenant un, deux, trois ou quatre atomes de chlore ; et de préférence aucun atome de fluor, un, deux ou trois atomes de fluor. De préférence, l'agent réducteur peut être un composé alcène en C₃ contenant quatre atomes d'halogène choisis parmi Cl et F ; au moins un étant un atome de chlore, de préférence au moins deux étant un atome de chlore. De manière davantage préférée, l'agent réducteur peut être un composé alcène en C₃ contenant quatre atomes d'halogène choisis parmi Cl et F ; parmi lesquels au plus trois atomes d'halogène sont des atomes de fluor, et au moins un est un atome de chlore.

De manière davantage préférée, l'agent réducteur peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb), le 1,1,1,3,3-pentachloropropane (HCC-240fa), le 1,1,3,3-tétrachloropropène (HCO-1230za), le 1,3,3,3-tétrachloropropène (HCO-1230zd), le 1-chloro-3,3,3-trifluoropropène (HCFO-1233zd), le 1,1,1,3-tétrachloropropane (HCC-250fb), le 1,1,3-trichloropropène (HCO-1240za), le 3,3,3-trichloropropène (HCO-1240zf).

En particulier, l'agent réducteur peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb).

La température de la seconde étape (ii) peut être d'environ 100 °C à environ 450 °C. La seconde étape (ii) peut être réalisée avec une durée de contact d'environ 1 à environ 100 s, de préférence de 1 à 75 s, de manière davantage préférée de 5 à 50 s ; pendant une durée supérieure à 1 heure, de préférence de 1 à 50 heures. De préférence, la température de la seconde étape (ii) peut être d'environ 300 à environ 400 °C, avec une durée de contact d'environ 1 à environ 100 s, de préférence de 1 à 75 s, de manière davantage préférée de 5 à 50 s ; pendant une durée supérieure à 1 heure, de préférence de 1 à 50 heures. En variante, la seconde étape (ii) peut être réalisée pendant une durée inférieure à 1 heure. En variante, la seconde étape (ii) peut être réalisée à une température allant de 200 à 300 °C. La seconde étape (ii) peut être réalisée à une pression allant de la pression atmosphérique à 5 bar.

Les étapes (i) et (ii) peuvent toutes les deux être répétées, jusqu'à ce que l'activité du catalyseur atteigne son meilleur niveau.

Selon un mode de réalisation particulier, avant le procédé d'activation tel que défini précédemment, le catalyseur peut être mis en contact avec un mélange comprenant HF et un composé chloré tel que défini dans le présent document. La mise en contact peut être réalisée pendant environ 6 à environ 100 heures (par exemple pendant moins de 50 heures). Le rapport molaire HF : composé chloré peut être d'environ 2 à environ 40. Le composé chloré peut être identique à celui utilisé ultérieurement pendant la réaction de fluoration à l'étape a). En variante, le composé chloré peut être différent du composé chloré qui est utilisé ultérieurement pendant la réaction de fluoration. Par conséquent, selon ce mode de réalisation particulier, l'étape d'activation peut être réalisée par :
(i') la mise en contact du catalyseur de fluoration avec un mélange comprenant HF et un composé chloré,
(ii') la mise en contact du catalyseur de fluoration obtenu à l'étape (i') avec un courant gazeux contenant un oxydant, et
(iii') éventuellement ou non, le traitement du catalyseur de fluoration obtenu après l'étape (ii') avec l'agent réducteur.

Chaque étape peut être répétée, jusqu'à ce que l'activité du catalyseur atteigne son meilleur niveau.

Selon un autre mode de réalisation, le procédé d'activation peut être réalisé par :
(i") la mise en contact du catalyseur de fluoration avec un mélange gazeux comprenant HF, un courant gazeux contenant un oxydant et un composé chloré, et
(ii") éventuellement ou non, la mise en contact du catalyseur de fluoration obtenu à l'étape (i") avec un agent réducteur.

L'oxydant, le composé chloré et l'agent réducteur sont tels que définis précédemment. La proportion d'oxydant dans le mélange d'HF, du composé chloré et de l'oxydant peut être d'environ 2 à environ 98 % en moles. La proportion de composé chloré dans le mélange d'HF, du composé chloré et de l'oxydant peut être d'environ 2 à environ 98 % en moles. La proportion d'HF dans le mélange d'HF, du composé chloré et de l'oxydant peut être d'environ 2 à environ 98 % en moles. Les conditions de procédé de l'étape d'activation sont définies précédemment. Chaque étape peut être répétée, jusqu'à ce que l'activité du catalyseur atteigne son meilleur niveau.

Les étapes (i), (ii), ou (i'), (ii'), (iii'), ou (i"), (ii") peuvent être répétées une, deux fois ou plus de manière alternée.

### Régénération du catalyseur

Les présents inventeurs ont également découvert que la présence de sous-produits peut être limitée par soumission du catalyseur à des étapes de régénération lors desquelles il est mis en contact avec un courant gazeux contenant un oxydant, puis avec un agent réducteur.

Selon un mode de réalisation préféré, la régénération du catalyseur de fluoration (étape b) du présent procédé comprend :
c) le traitement dudit catalyseur de fluoration avec un courant gazeux contenant un oxydant pour former un catalyseur de fluoration oxydé ; et
d) le traitement dudit catalyseur de fluoration oxydé obtenu à l'étape c) avec un mélange gazeux comprenant un agent réducteur.

Selon un mode de réalisation, l'oxydant utilisé à l'étape c) est l'oxygène ou l'air ou un mélange oxygène/azote ou le chlore. Lorsque l'étape c) est réalisée avec de l'air ou un mélange oxygène/azote, la proportion d'oxygène peut être de 20 à environ 100 % en moles par rapport au mélange d'oxygène plus azote.

Selon un autre mode de réalisation, l'étape c) peut être réalisée avec de l'oxygène ou de l'air ou un mélange oxygène/azote ou du chlore et HF. La proportion d'oxygène peut être d'environ 2 à environ 98 % en moles par rapport au mélange d'oxygène plus HF, et d'environ 20 à environ 100 % en moles, par rapport au mélange d'oxygène plus azote.

La température pendant l'étape c) peut aller de 250 à 500 °C, de préférence de 300 à 450 °C, de manière davantage préférée de 350 à 400 °C ou de 325°C à 375°C ; avec une durée de contact de 1 à 200 s, de préférence de 1 à 150 s, de manière davantage préférée de 5 à 100 s ; et pendant une durée de 1 à environ 1 500 heures, de préférence de 2 à 1 000 heures, de manière davantage préférée de 4 à 500 heures, de manière préférée entre toutes de 10 à 200 heures, en particulier de 15 à 150 heures, plus particulièrement de 15 à 70 heures. L'étape c) peut être réalisée à une pression allant de la pression atmosphérique jusqu'à 20 bar, avantageusement de la pression atmosphérique jusqu'à 5 bar, de préférence de la pression atmosphérique jusqu'à 3 bar. Selon un mode de réalisation préféré, la température pendant l'étape c) peut être d'environ 250 à 500 °C, avec une durée de contact d'environ 1 à 200 s, pendant une durée de 10 à 200 heures et à une pression allant de la pression atmosphérique à 20 bar, de préférence de la pression atmosphérique jusqu'à 3 bar. Selon un mode de réalisation particulièrement préféré, la température pendant l'étape c) peut être d'environ 325 à 375 °C, avec une durée de contact d'environ 5 à 100 s, pendant une durée de 15 à 75 heures et à une pression allant de la pression atmosphérique à 20 bar, de préférence de la pression atmosphérique jusqu'à 3 bar.

Le mélange gazeux utilisé à l'étape (d) peut comprendre un gaz inerte, en particulier lorsque l'agent réducteur est un hydrohalocarbure en C₁-C₁₀, de préférence un composé chloré. Le gaz inerte peut être l'azote, l'hélium, l'argon, HF ou leurs mélanges. Le gaz inerte peut être l'azote, l'hélium, l'argon ou leurs mélanges. En particulier, le gaz inerte peut être un mélange d'HF et d'azote. Plus particulièrement, le gaz inerte peut être un mélange d'HF et d'azote lorsque l'agent réducteur est un hydrohalocarbure en C₁-C₁₀, avantageusement un hydrohalocarbure en C₃, de préférence un alcane ou un alcène en C3 comportant au moins un atome de chlore.

Selon un mode de réalisation préféré, le mélange gazeux de l'étape (d) comprend de 1 à 10 % en volume d'agent réducteur, de préférence de 2 à 9 % en volume, de manière davantage préférée de 3 à 7 % en volume, par rapport au volume total du mélange gazeux.

Selon un mode de réalisation préféré, l'étape d) est réalisée avec un agent réducteur choisi dans le groupe constitué par les hydrohalocarbures en C₁-C₁₀. En particulier, l'agent réducteur peut être un composé chloré tel que défini précédemment. Plus particulièrement, l'agent réducteur peut être l'hydrogène ou un alcane ou un alcène en C₃ comportant au moins un atome de chlore.

Par conséquent, l'agent réducteur peut être un composé alcane en C₃ contenant au moins un atome de chlore, de préférence au moins deux atomes de chlore ; et de préférence de 0 à 5 atomes de fluor. L'agent réducteur peut être un composé alcane en C₃ contenant un, deux, trois, quatre, cinq ou six atomes de chlore ; et de préférence aucun atome de fluor, un, deux, trois, quatre ou cinq atomes de fluor. De préférence, l'agent réducteur peut être un composé alcane en C₃ contenant quatre ou cinq atomes d'halogène choisis parmi Cl et F ; au moins un étant un atome de chlore, de préférence au moins deux étant un atome de chlore. De manière davantage préférée, l'agent réducteur peut être un composé alcane en C₃ contenant quatre ou cinq atomes d'halogène choisis parmi Cl et F, parmi lesquels au plus quatre atomes d'halogène sont des atomes de fluor, et au moins un est un atome de chlore, de préférence au moins deux sont un atome de chlore.

De préférence, l'agent réducteur peut être un composé alcène en C₃ contenant au moins un atome de chlore, de préférence au moins deux atomes de chlore ; et de préférence de 0 à 3 atomes de fluor. L'agent réducteur peut être un composé alcène en C₃ contenant un, deux, trois ou quatre atomes de chlore ; et de préférence aucun atome de fluor, un, deux ou trois atomes de fluor. De préférence, l'agent réducteur peut être un composé alcène en C₃ contenant quatre atomes d'halogène choisis parmi Cl et F ; au moins un étant un atome de chlore, de préférence au moins deux étant un atome de chlore. De manière davantage préférée, l'agent réducteur peut être un composé alcène en C₃ contenant quatre atomes d'halogène choisis parmi Cl et F, parmi lesquels au plus trois atomes d'halogène sont des atomes de fluor, et au moins un est un atome de chlore.

De manière davantage préférée, l'agent réducteur peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb), le 1,1,1,3,3-pentachloropropane (HCC-240fa), le 1,1,3,3-tétrachloropropène (HCO-1230za), le 1,3,3,3-tétrachloropropène (HCO-1230zd), le 1-chloro-3,3,3-trifluoropropène (HCFO-1233zd), le 1,1,1,3-tétrachloropropane (HCC-250fb), le 1,1,3-trichloropropène (HCO-1240za), le 3,3,3-trichloropropène (HCO-1240zf).

En particulier, l'agent réducteur peut être choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb).

L'étape d) peut être réalisée à une température allant d'environ 100 °C à environ 450 °C. De préférence, l'étape d) peut être réalisée à une température de 300 à 400 °C, en particulier de 325°C à 375°C. L'étape d) peut être réalisée pendant une durée de contact d'environ 1 à environ 100 s, de préférence de 1 à 75 s, de manière davantage préférée de 5 à 50 s. De préférence, l'étape d) peut être réalisée pendant une durée supérieure à 1 heure, de préférence de 1 à 50 heures, en particulier de 1 à 15 heures. De manière davantage préféré, l'étape d) peut être réalisée à une température de 300 à 400 °C, en particulier de 325°C à 375°C, avec une durée de contact d'environ 1 à environ 100 s, de préférence de 1 à 75 s, de manière davantage préférée de 5 à 50 s ; pendant une durée supérieure à 1 heure, de préférence de 1 à 50 heures, en particulier de 1 à 15 heures. En variante, l'étape d) peut être réalisée pendant une durée inférieure à 1 heure. En variante, l'étape d) peut être réalisée à une température allant de 200 à 300 °C. L'étape d) peut être réalisée à une pression allant de la pression atmosphérique à 5 bar.

Les étapes c) et d) peuvent toutes les deux être répétées une, deux fois ou plus de manière alternée. En particulier, l'étape d) peut être répétée indépendamment une, deux fois ou plus, c.-à-d. que chaque étape d) peut être répétée avec différents agent réducteurs. Par exemple, l'étape d) peut être réalisée avec de l'hydrogène et peut être répétée avec le 2-chloro-3,3,3-trifluoro-1-propène.

### Exemples

Les exemples suivants illustrent l'invention sans la limiter.

L'équipement comprend un réacteur tubulaire en alliage INCONEL® 600 ayant un diamètre interne de 21 mm et un dispositif de préchauffage par résistance de 12 m ayant un diamètre interne de 6 mm. Le système est immergé dans un bain de sable fluidisé. Le réacteur est équipé de dispositifs de contrôle de la pression et de la température. Les réactifs, mélangés au préalable, sont introduits au fond du réacteur.

Le courant gazeux sortant du réacteur traverse un épurateur à eau avant d'être séché, échantillonné et analysé par chromatographie gazeuse. Un CG HP de modèle 5890 est utilisé pour toutes les expériences. Le chromatographe est équipé d'une colonne RTX®-200 et d'une colonne ShinCarbon (Restek), toutes les deux connectées à un détecteur de conductivité thermique (TCD).

### Exemple comparatif 1 : Fluoration d'HFCO-1233xf : régénération du catalyseur de fluoration sans traitement avec un mélange gazeux comprenant un agent réducteur.

L'équipement décrit ci-dessus est utilisé pour réaliser la fluoration catalytique en phase vapeur d'HFCO-1233xf. Le réacteur est chargé avec environ 130 cm³ d'un catalyseur en vrac de chrome commercial activé au préalable.

La réaction est réalisée à une pression absolue constante de P = 5 bar et la température est maintenue à T = 380 °C. Du fluorure d'hydrogène anhydre (HF), HFCO-1233xf et de l'air sont introduits en continu dans le réacteur. Le rapport molaire entre HF et HFCO-1233xf est de 20. Le rapport molaire entre l'oxygène (O₂) et HFCO-1233xf est de 0,04. La durée de contact est calculée comme étant de 20 secondes dans les conditions de réaction.

Après 70 heures de réaction, alors que la conversion d'HFCO-1233xf est d'environ 20 %, la réaction est arrêtée et une étape de régénération est réalisée avec un traitement à l'air pendant 72 heures à 13 l/h, T = 380 °C et à pression atmosphérique.

La réaction est ensuite redémarrée en utilisant les mêmes conditions.

Le produit majeur obtenu autre qu'HFO-1234yf est HFC-245cb. Ce composé peut être recyclé et réutilisé, et est considéré comme une substance utile. Les sous-produits obtenus et leurs sélectivités sont indiqués dans le tableau 1 ci-dessous :

**Tableau 1**

| | Sélectivités | | | | | |
|---|---|---|---|---|---|---|
| Temps (h) | CO | F143a | F1234yf + F245cb | F1234zeZ + F245fa | F1233zdE | Autres |
| 6 | 2,26 | 0,56 | 86,17 | 2,78 | 2,16 | 2,24 |
| 12 | 2,13 | 0,54 | 87,22 | 2,53 | 1,89 | 2,09 |
| 24 | 2,00 | 0,46 | 89,61 | 1,83 | 1,47 | 1,58 |
| 30 | 2,30 | 0,45 | 89,05 | 1,74 | 1,34 | 1,72 |
| 36 | 2,06 | 0,45 | 89,90 | 1,60 | 1,15 | 1,67 |

### Exemple 2 selon la présente invention : Fluoration d'HFCO-1233xf : régénération du catalyseur de fluoration avec traitement avec un mélange gazeux comprenant un agent réducteur.

L'équipement décrit ci-dessus est utilisé pour réaliser la fluoration catalytique en phase vapeur d'HFCO-1233xf. Le réacteur est chargé avec environ 130 cm³ d'un catalyseur en vrac de chrome commercial activé au préalable.

La réaction est réalisée à une pression absolue constante de P = 5 bar et la température est maintenue à T = 380 °C. Du fluorure d'hydrogène anhydre (HF), HFCO-1233xf et de l'air sont introduits en continu dans le réacteur. Le rapport molaire entre HF et HFCO-1233xf est de 20. Le rapport molaire entre l'oxygène (O₂) et HFCO-1233xf est de 0,04. La durée de contact est calculée comme étant de 20 secondes dans les conditions de réaction.

Après 76 heures de réaction, alors que la conversion d'HFCO-1233xf est d'environ 35 %, la réaction est arrêtée et une étape de régénération est réalisée
- avec un traitement à l'air pendant 72 heures à 5 l/h, T = 380 °C et à pression atmosphérique,
- avec un traitement utilisant un mélange d'HFCO-1233xf : HF : azote (14,1 g/h, 43,1 g/h ; 51 l/h) pendant 5 heures à T = 350 °C.

La réaction est ensuite redémarrée en utilisant les mêmes conditions.

Le produit majeur obtenu autre qu'HFO-1234yf est HFC-245cb. Ce composé peut être recyclé et réutilisé, et est considéré comme une substance utile. Les sous-produits obtenus et leurs sélectivités sont indiqués dans le tableau 2 ci-dessous :

**Tableau 2**

| | Sélectivités | | | | | |
|---|---|---|---|---|---|---|
| Temps (h) | CO | F143a | F1234yf + F245cb | F1234zeZ + F245fa | F1233zdE | Autres |
| 6 | 2,05 | 0,45 | 89,32 | 1,69 | 1,53 | 0,26 |
| 12 | 1,99 | 0,45 | 89,70 | 1,60 | 1,40 | 0,27 |
| 24 | 1,90 | 0,40 | 90,66 | 1,39 | 1,21 | 0,31 |
| 30 | 2,01 | 0,42 | 90,23 | 1,46 | 1,15 | 0,35 |
| 36 | 2,02 | 0,42 | 90,28 | 1,41 | 1,11 | 0,36 |

### Exemple comparatif 3 : Fluoration d'HFCO-1233xf : régénération du catalyseur de fluoration sans traitement avec un mélange gazeux comprenant un agent réducteur.

L'équipement décrit ci-dessus est utilisé pour réaliser la fluoration catalytique en phase vapeur d'HFCO-1233xf. Le réacteur est chargé avec environ 130 cm³ d'un catalyseur en vrac de chrome commercial activé au préalable.

La réaction est réalisée à une pression absolue constante de P = 5 bar et la température est maintenue à T = 350°C. Du fluorure d'hydrogène anhydre (HF), HFCO-1233xf et de l'air sont introduits en continu dans le réacteur. Le rapport molaire entre HF et HFCO-1233xf est de 20. Le rapport molaire entre l'oxygène (O₂) et HFCO-1233xf est de 0,04. La durée de contact est calculée comme étant de 34 secondes dans les conditions de réaction.

Après 48 heures de réaction, les débits des réactifs sont augmentés et la nouvelle durée de contact est calculée comme étant de 20 secondes dans les conditions de réaction. Après 24 heures de réaction supplémentaires, alors que la conversion d'HFCO-1233xf est d'environ 45 %, la réaction est arrêtée et une étape de régénération est réalisée avec un traitement à l'air pendant 72 heures à 7,5 l/h, T = 350 °C et à pression atmosphérique.

La réaction est ensuite redémarrée en utilisant les mêmes conditions (durée de contact calculée comme étant de 20 secondes dans les conditions de réaction).

Le produit majeur obtenu autre qu'HFO-1234yf est HFC-245cb. Ce composé peut être recyclé et réutilisé, et est considéré comme une substance utile. Les sous-produits obtenus et leurs sélectivités sont indiqués dans le tableau 3 ci-dessous :

**Tableau 3**

| | Sélectivités | | | | |
|---|---|---|---|---|---|
| Temps (h) | F1234yf + F245cb | F1234zeE + F1243zf | F1234zeZ + F245fa | F1224xe | F1233zdE |
| 6 | 92,41 | 0,95 | 1,25 | 0,33 | 0,89 |
| 12 | 93,04 | 0,99 | 1,40 | 0,13 | 1,08 |
| 18 | 94,18 | 0,80 | 1,16 | 0,08 | 0,80 |

### Exemple 4: Fluoration d'HFCO-1233xf : régénération du catalyseur de fluoration avec traitement avec un mélange gazeux comprenant un agent réducteur.

L'équipement décrit ci-dessus est utilisé pour réaliser la fluoration catalytique en phase vapeur d'HFCO-1233xf. Le réacteur est chargé avec environ 130 cm³ d'un catalyseur en vrac de chrome commercial activé au préalable.

La réaction est réalisée à une pression absolue constante de P = 5 bar et la température est maintenue à T = 350 °C. Du fluorure d'hydrogène anhydre (HF), HFCO-1233xf et de l'air sont introduits en continu dans le réacteur. Le rapport molaire entre HF et HFCO-1233xf est de 20. Le rapport molaire entre l'oxygène (O₂) et HFCO-1233xf est de 0,04. La durée de contact est calculée comme étant de 20 secondes dans les conditions de réaction.

Après 48 heures de réaction, alors que la conversion d'HFCO-1233xf est d'environ 61 %, la réaction est arrêtée et une étape de régénération est réalisée
- avec un traitement à l'air pendant 48 heures à 5 l/h, T = 350 °C et à pression atmosphérique,
- avec un traitement utilisant un mélange d'hydrogène : azote (1,25 l/h ; 25 l/h) pendant 5 heures à T = 325 °C.
- avec un traitement à l'azote pendant 24 heures à 10 l/h, T = 350 °C et à une pression absolue P = 1,6 bar.

La réaction est ensuite redémarrée en utilisant les mêmes conditions.

Le produit majeur obtenu autre qu'HFO-1234yf est HFC-245cb. Ce composé peut être recyclé et réutilisé, et est considéré comme une substance utile. Les sous-produits obtenus et leurs sélectivités sont indiqués dans le tableau 4 ci-dessous :

**Tableau 4**

| | Sélectivités | | | | |
|---|---|---|---|---|---|
| Temps (h) | F1234yf + F245cb | F1234zeE + F1243zf | F1234zeZ + F245fa | F1224xe | F1233zdE |
| 6 | 95,63 | 0,51 | 0,67 | 0,07 | 0,44 |
| 18 | 95,01 | 0,57 | 0,78 | 0,07 | 0,47 |

## Revendications

1. Procédé de fluoration d'un composé chloré alcane ou alcène en C₃ contenant au moins un atome de chlore en un composé fluoré alcane ou alcène en C₃ contenant au moins un atome de fluor, comprenant les étapes suivantes :
(a) la mise en contact, dans un réacteur, du composé chloré avec du fluorure d'hydrogène en phase gazeuse en présence d'un catalyseur de fluoration pour produire un composé fluoré, le nombre d'atomes de fluor dans le composé fluoré est supérieur au nombre d'atomes de fluor dans le composé chloré et
(b) la régénération du catalyseur de fluoration utilisé à l'étape a),
l'étape (b) de régénération du catalyseur de fluoration comprenant (c) le traitement dudit catalyseur de fluoration avec un courant gazeux contenant un oxydant pour former un catalyseur de fluoration oxydé, et (d) le traitement du catalyseur de fluoration oxydé obtenu à l'étape (c) avec un mélange gazeux comprenant un agent réducteur et un gaz inerte ; le catalyseur régénéré à l'étape b) étant réutilisé à l'étape a) ; et-l'agent réducteur étant choisi dans le groupe constitué par les hydrohalocarbures en C₁-C₁₀; ledit catalyseur étant sélectionné parmi le groupe consistant en l'oxyfluorure de chrome, des oxydes de chrome, des halogénures de chrome et leurs mélanges.

2. Procédé selon l'une quelconque des revendications précédentes, dans lequel le gaz inerte est choisi parmi l'azote, l'hélium, l'argon, leurs mélanges.

3. Procédé selon la revendication précédente **caractérisé en ce que** lorsque l'agent réducteur est un hydrohalocarbure en C₁-C₁₀, le mélange gazeux comprend de l'HF.

4. Procédé selon l'une quelconque des revendications précédentes, dans lequel le mélange gazeux de l'étape (d) comprend de 1 à 10 % en volume d'agent réducteur, de préférence de 2 à 9 % en volume, de manière davantage préférée de 3 à 7 % en volume, par rapport au volume total du mélange gazeux.

5. Procédé selon l'une quelconque des revendications précédentes **caractérisé en ce que** le mélange gazeux de l'étape (d) comprend un hydrohalocarbures en C₂-C₆, de l'azote ou de l'argon, et de l'HF, de préférence le mélange gazeux de l'étape (d) comprend un hydrohalocarbure en C3, de l'azote et de l'HF.

6. Procédé selon l'une quelconque des revendications précédentes, dans lequel l'étape d) est réalisée à une température allant de 100 °C à 450 °C, avec une durée de contact de 1 à 100 s, de préférence de 1 à 75 s, de manière davantage préférée de 5 à 50 s, pendant une durée supérieure à 1 heure, de préférence de 1 à 50 heures, en particulier de 4 à 25 heures.

7. Procédé selon l'une quelconque des revendications précédentes, dans lequel le composé chloré est le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf), le 2-chloro-1,1,1,2-tétrafluoropropane (HCFC-244bb), le 1,1,1,3,3-pentachloropropane (HCC-240fa), le 1,1,3,3-tétrachloropropène (HCO-1230za), le 1,3,3,3-tétrachloropropène (HCO-1230zd), le 1-chloro-3,3,3-trifluoropropène (HCFO-1233zd), le 1,1,1,3-tétrachloropropane (HCC-250fb), le 1,1,3-trichloropropène (HCO-1240za), le 3,3,3-trichloropropène (HCO-1240zf) ; de manière davantage préférée, le composé chloré est choisi dans le groupe constitué par le 2-chloro-3,3,3-trifluoro-1-propène (HFCO-1233xf), le 1,1,1,2,3-pentachloropropane (HCC-240db), le 1,1,2,2,3-pentachloropropane (HCC-240aa), le 2,3-dichloro-1,1,1-trifluoropropane (HCFC-243db), le 1,1,2,3-tétrachloro-1-propène (HCO-1230xa), le 2,3,3,3-tétrachloro-1-propène (HCO-1230xf) ou les 2-chloro-1,1,1,2-tétrafluorocarbures (HCFC-244bb).

8. Procédé selon l'une quelconque des revendications précédentes, dans lequel le composé fluoré est le 2,3,3,3-tétrafluoro-1-propène (HFO-1234yf), le 2-chloro-1,1,1,2-tétrafluoropropane (HCFC-244bb), le 1,1,1,2,2-pentafluoropropane (HFC-245cb), le 1,1,1,3,3-pentafluoropropane (HFC-245fa), le 1,1,1,3-tétrafluoro-3-chloropropane (HCFC-244fa), le 1-chloro-3,3,3-trifluoropropène (HCFO-1233zd), le 1,3,3,3-tétrafluoropropène (HFO-1234ze), le 3,3,3-trifluoropropène (HFO-1243zf) ou le 2-chloro-3,3,3-trifluoro-1-propène (HFCO-1233xf).

9. Procédé selon l'une quelconque des revendications précédentes, dans lequel la fluoration du composé chloré en un composé fluoré est :
- 2-chloro-3,3,3-trifluoro-1-propène (HFCO-1233xf) en 2,3,3,3-tétrafluoro-1-propène (HFO-1234yf) ;
- 1,1,1,2,3-pentachloropropane (HCC-240db) en 2,3,3,3-tétrafluoro-1-propène (HFO-1234yf) ;
- 1,1,2,2,3-pentachloropropane (HCC-240aa) en 2,3,3,3-tétrafluoro-1-propène (HFO-1234yf) ;
- 2,3-dichloro-1,1,1-trifluoropropane (HCFC-243db) en 2,3,3,3-tétrafluoro-1-propène (HFO-1234yf) ;
- 1,1,2,3-tétrachloro-1-propène (HCO-1230xa) en 2,3,3,3-tétrafluoro-1-propène (HFO-1234yf) ;
- 2,3,3,3-tétrachloro-1-propène (HCO-1230xf) en 2,3,3,3-tétrafluoro-1-propène (HFO-1234yf) ;
- 1,1,1,2,3-pentachloropropane (HCC-240db) en 2-chloro-3,3,3-trifluoro-1-propène (HFCO-1233xf) ;
- 1,1,2,2,3-pentachloropropane (HCC-240aa) en 2-chloro-3,3,3-trifluoro-1-propène (HFCO-1233xf) ;
- 1,1,2,3-tétrachloro-1-propène (HCO-1230xa) en 2-chloro-3,3,3-trifluoro-1-propène (HFCO-1233xf) ;
- 2,3,3,3-tétrachloro-1-propène (HCO-1230xf) en 2-chloro-3,3,3-trifluoro-1-propène (HFCO-1233xf) ;
- 2-chloro-3,3,3-trifluoro-1-propène (HFCO-1233xf) en 2-chloro-1,1,1,2-tétrafluoropropane (HCFC-244bb) ;
- 1,1,1,2,3-pentachloropropane (HCC-240db) en 2-chloro-1,1,1,2-tétrafluoropropane (HCFC-244bb) ;
- 1,1,2,2,3-pentachloropropane (HCC-240aa) en 2-chloro-1,1,1,2-tétrafluoropropane (HCFC-244bb) ;
- 2,3-dichloro-1,1,1-trifluoropropane (HCFC-243db) en 2-chloro-1,1,1,2-tétrafluoropropane (HCFC-244bb) ;
- 1,1,2,3-tétrachloro-1-propène (HCO-1230xa) en 2-chloro-1,1,1,2-tétrafluoropropane (HCFC-244bb) ;
- 2,3,3,3-tétrachloro-1-propène (HCO-1230xf) en 2-chloro-1,1,1,2-tétrafluoropropane (HCFC-244bb) ;
- 1,1,1,2,3-pentachloropropane (HCC-240db) en 1,1,1,2,2-pentafluoropropane (HFC-245cb) ;
- 1,1,2,2,3-pentachloropropane (HCC-240aa) en 1,1,1,2,2-pentafluoropropane (HFC-245cb) ;
- 2,3-dichloro-1,1,1-trifluoropropane (HCFC-243db) en 1,1,1,2,2-pentafluoropropane (HFC-245cb) ;
- 1,1,2,3-tétrachloro-1-propène (HCO-1230xa) en 1,1,1,2,2-pentafluoropropane (HFC-245cb) ;
- 2,3,3,3-tétrachloro-1-propène (HCO-1230xf) en 1,1,1,2,2-pentafluoropropane (HFC-245cb).

10. Procédé selon les revendications précédentes, dans lequel le catalyseur de fluoration contient un ou plusieurs co-catalyseurs comprenant un sel d'un métal de transition choisi dans le groupe constitué par Co, Zn, Mn, Mg, V, Mo, Te, Nb, Sb, Ta, Ni ; ou un sel phosphoreux.

11. Procédé selon l'une quelconque des revendications précédentes, comprenant une étape d'activation réalisée avant l'étape (a) qui comprend une première étape de mise en contact du catalyseur de fluoration avec un courant gazeux contenant un oxydant sélectionné parmi l'oxygène, l'air, le chlore ou un mélange d'oxygène et d'azote.

12. Procédé selon la revendication précédente, dans lequel l'étape d'activation comprend une seconde étape, après la première étape, de traitement du catalyseur de fluoration obtenu après la première étape avec un mélange gazeux comprenant un agent réducteur et un gaz inerte, ledit agent réducteur étant l'hydrogène ou un hydrohalocarbure en C₂-C₆..

13. Procédé selon l'une quelconque des revendications précédentes, dans lequel les étapes (a) et (b) sont réalisées en alternance.

14. Procédé selon l'une quelconque des revendications précédentes, dans lequel une purge du réacteur est réalisée avant et/ou après l'étape (b), la purge étant de préférence réalisée par introduction d'un courant d'azote dans le réacteur ou en maintenant le réacteur sous vide.

15. Procédé de fluoration d'un 2,3,3,3-tétrafluoropropène en 1,1,1,2,2-pentafluoropropane, comprenant les étapes suivantes :
(c) la mise en contact, dans un réacteur, de 2,3,3,3-tétrafluoropropène avec du fluorure d'hydrogène en phase gazeuse en présence d'un catalyseur de fluoration pour produire le 1,1,1,2,2-pentafluoropropane, et
(d) la régénération du catalyseur de fluoration utilisé à l'étape a),
l'étape (b) de régénération du catalyseur de fluoration comprenant (c) le traitement dudit catalyseur de fluoration avec un courant gazeux contenant un oxydant pour former un catalyseur de fluoration oxydé, et (d) le traitement du catalyseur de fluoration oxydé obtenu à l'étape (c) avec un mélange gazeux comprenant un agent réducteur ; le catalyseur régénéré à l'étape b) étant réutilisé à l'étape a) ; l'agent réducteur étant choisi dans le groupe constitué par les hydrohalocarbures en C₁-C₁₀; ledit catalyseur étant sélectionné parmi le groupe consistant en l'oxyfluorure de chrome, des oxydes de chrome, des halogénures de chrome et leurs mélanges.

## Patentansprüche

1. Verfahren zur Fluorierung einer chlorierten C₃-Alkan- oder -Alkenverbindung, die mindestens ein Chloratom enthält, zu einer fluorierten C₃-Alkan- oder - Alkenverbindung, die mindestens ein Fluoratom enthält, das die folgenden Schritte umfasst:
(a) Inkontaktbringen der chlorierten Verbindung mit Fluorwasserstoff in der Gasphase in Gegenwart eines Fluorierungskatalysators in einem Reaktor zur Herstellung einer fluorierten Verbindung, wobei die Zahl der Fluoratome in der fluorierten Verbindung größer ist als die Zahl der Fluoratome in der chlorierten Verbindung, und
(b) Regenerieren des in Schritt a) verwendeten Fluorierungskatalysators,
wobei der Schritt (b) des Regenerierens des Fluorierungskatalysators (c) das Behandeln des Fluorierungskatalysators mit einem ein Oxidationsmittel enthaltenden Gasstrom zur Bildung eines oxidierten Fluorierungskatalysators und (d) das Behandeln des in Schritt (c) erhaltenen oxidierten Fluorierungskatalysators mit einer Gasmischung, die ein Reduktionsmittel und ein Inertgas umfasst, umfasst, wobei der in Schritt b) regenerierte Katalysator in Schritt a) wiederverwendet wird, wobei das Reduktionsmittel aus der Gruppe bestehend aus C₁-C₁₀-Halogenkohlenwasserstoffen ausgewählt wird und wobei der Katalysator aus der Gruppe bestehend aus Chromoxidfluorid, Chromoxiden, Chromhalogeniden und Mischungen davon ausgewählt wird.

2. Verfahren nach einem der vorhergehenden Ansprüche, wobei das Inertgas aus Stickstoff, Helium, Argon und Mischungen davon ausgewählt wird.

3. Verfahren nach einem der vorhergehenden Ansprüche, **dadurch gekennzeichnet, dass** dann, wenn es sich bei dem Reduktionsmittel um einen C₁-C₁₀-Halogenkohlenwasserstoff handelt, die Gasmischung HF umfasst.

4. Verfahren nach einem der vorhergehenden Ansprüche, wobei die Gasmischung von Schritt (d) 1 bis 10 Vol.-% Reduktionsmittel, vorzugsweise 2 bis 9 Vol.-%, weiter bevorzugt 3 bis 7 Vol.-%, bezogen auf das Gesamtvolumen der Gasmischung, umfasst.

5. Verfahren nach einem der vorhergehenden Ansprüche, **dadurch gekennzeichnet, dass** die Gasmischung von Schritt (d) einen C₂-C₆-Halogenkohlenwasserstoff, Stickstoff oder Argon und HF umfasst, vorzugsweise die Gasmischung von Schritt (d) einen C3-Halogenkohlenwasserstoff, Stickstoff und HF umfasst.

6. Verfahren nach einem der vorhergehenden Ansprüche, wobei Schritt d) bei einer Temperatur im Bereich von 100 °C bis 450 °C mit einer Kontaktdauer von 1 bis 100 s, vorzugsweise von 1 bis 75 s, weiter bevorzugt von 5 bis 50 s, über eine Dauer von mehr als 1 Stunde, vorzugsweise von 1 bis 50 Stunden, insbesondere von 4 bis 25 Stunden, durchgeführt wird.

7. Verfahren nach einem der vorhergehenden Ansprüche, wobei es sich bei der chlorierten Verbindung um 2-Chlor-3,3,3-trifluor-1-propen (HFCO-1233xf), 1,1,1,2,3-Pentachlorpropan (HCC-240db), 1,1,2,2,3-Pentachlorpropan (HCC-240aa), 2,3-Dichlor-1,1,1-trifluorpropan (HCFC-243db), 1,1,2,3-Tetrachlor-1-propen (HCO-1230xa), 2,3,3,3-Tetrachlor-1-propen (HCO-1230xf), 2-Chlor-1,1,1,2-tetrafluorpropan (HCFC-244bb), 1,1,1,3,3-Pentachlorpropan (HCC-240fa), 1,1,3,3-Tetrachlorpropen (HCO-1230za), 1,3,3,3-Tetrachlorpropen (HCO-1230zd), 1-Chlor-3,3,3-trifluorpropen (HCFO-1233zd), 1,1,1,3-Tetrachlorpropan (HCC-250fb), 1,1,3-Trichlorpropen (HCO-1240za), 3,3,3-Trichlorpropen (HCO-1240zf) handelt; weiter bevorzugt die chlorierte Verbindung aus der Gruppe bestehend aus 2-Chlor-3,3,3-trifluor-1-propen (HFCO-1233xf), 1,1,1,2,3-Pentachlorpropan (HCC-240db), 1,1,2,2,3-Pentachlorpropan (HCC-240aa), 2,3-Dichlor-1,1,1-trifluorpropan (HCFC-243db), 1,1,2,3-Tetrachlor-1-propen (HCO-1230xa), 2,3,3,3-Tetrachlor-1-propen (HCO-1230xf) oder 2-Chlor-1,1,1,2-tetrafluorkohlenstoffen (HCFC-244bb) ausgewählt ist.

8. Verfahren nach einem der vorhergehenden Ansprüche, wobei es sich bei der fluorierten Verbindung um 2,3,3,3-Tetrafluor-1-propen (HFO-1234yf), 2-Chlor-1,1,1,2-tetrafluorpropan (HCFC-244bb), 1,1,1,2,2-Pentafluorpropan (HFC-245cb), 1,1,1,3,3-Pentafluorpropan (HFC-245fa), 1,1,1,3-Tetrafluor-3-chlorpropan (HCFC-244fa), 1-Chlor-3,3,3-trifluorpropen (HCFO-1233zd), 1,3,3,3-Tetrafluorpropen (HFO-1234ze), 3,3,3-Trifluorpropen (HFO-1243zf) oder 2-Chlor-3,3,3-trifluor-1-propen (HFCO-1233xf) handelt.

9. Verfahren nach einem der vorhergehenden Ansprüche, wobei es sich bei der Fluorierung der chlorierten Verbindung zu einer Fluorverbindung um Folgendes handelt:
- 2-Chlor-3,3,3-trifluor-1-propen (HFCO-1233xf) zu 2,3,3,3-Tetrafluor-1-propen (HFO-1234yf);
- 1,1,1,2,3-Pentachlorpropan (HCC-240db) zu 2,3,3,3-Tetrafluor-1-propen (HFO-1234yf);
- 1,1,2,2,3-Pentachlorpropan (HCC-240aa) zu 2,3,3,3-Tetrafluor-1-propen (HFO-1234yf);
- 2,3-Dichlor-1,1,1-trifluorpropan (HCFC-243db) zu 2,3,3,3-Tetrafluor-1-propen (HFO-1234yf);
- 1,1,2,3-Tetrachlor-1-propen (HCO-1230xa) zu 2,3,3,3-Tetrafluor-1-propen (HFO-1234yf);
- 2,3,3,3-Tetrachlor-1-propen (HCO-1230xf) zu 2,3,3,3-Tetrafluor-1-propen (HFO-1234yf);
- 1,1,1,2,3-Pentachlorpropan (HCC-240db) zu 2-Chlor-3,3,3-trifluor-1-propen (HFCO-1233xf);
- 1,1,2,2,3-Pentachlorpropan (HCC-240aa) zu 2-Chlor-3,3,3-trifluor-1-propen (HFCO-1233xf);
- 1,1,2,3-Tetrachlor-1-propen (HCO-1230xa) zu 2-Chlor-3,3,3-trifluor-1-propen (HFCO-1233xf);
- 2,3,3,3-Tetrachlor-1-propen (HCO-1230xf) zu 2-Chlor-3,3,3-trifluor-1-propen (HFCO-1233xf);
- 2-Chlor-3,3,3-trifluor-1-propen (HFCO-1233xf) zu 2-Chlor-1,1,1,2-tetrafluorpropan (HCFC-244bb);
- 1,1,1,2,3-Pentachlorpropan (HCC-240db) zu 2-Chlor-1,1,1,2-tetrafluorpropan (HCFC-244bb);
- 1,1,2,2,3-Pentachlorpropan (HCC-240aa) zu 2-Chlor-1,1,1,2-tetrafluorpropan (HCFC-244bb);
- 2,3-Dichlor-1,1,1-trifluorpropan (HCFC-243db) zu 2-Chlor-1,1,1,2-tetrafluorpropan (HCFC-244bb);
- 1,1,2,3-Tetrachlor-1-propen (HCO-1230xa) zu 2-Chlor-1,1,1,2-tetrafluorpropan (HCFC-244bb);
- 2,3,3,3-Tetrachlor-1-propen (HCO-1230xf) zu 2-Chlor-1,1,1,2-tetrafluorpropan (HCFC-244bb);
- 1,1,1,2,3-Pentachlorpropan (HCC-240db) zu 1,1,1,2,2-Pentafluorpropan (HFC-245cb);
- 1,1,2,2,3-Pentachlorpropan (HCC-240aa) zu 1,1,1,2,2-Pentafluorpropan (HFC-245cb);
- 2,3-Dichlor-1,1,1-trifluorpropan (HCFC-243db) zu 1,1,1,2,2-Pentafluorpropan (HFC-245cb);
- 1,1,2,3-Tetrachlor-1-propen (HCO-1230xa) zu 1,1,1,2,2-Pentafluorpropan (HFC-245cb);
- 2,3,3,3-Tetrachlor-1-propen (HCO-1230xf) zu 1,1,1,2,2-Pentafluorpropan (HFC-245cb).

10. Verfahren nach einem der vorhergehenden Ansprüche, wobei der Fluorierungskatalysator einen oder mehrere Cokatalysatoren enthält, die ein Salz eines Übergangsmetalls aus der Gruppe bestehend aus Co, Zn, Mn, Mg, V, Mo, Te, Nb, Sb, Ta, Ni oder ein phosphorhaltiges Salz umfassen.

11. Verfahren nach einem der vorhergehenden Ansprüche, umfassend einen vor Schritt (a) durchgeführten Aktivierungsschritt, der einen ersten Schritt des Inkontaktbringens des Fluorierungskatalysators mit einem ein aus Sauerstoff, Luft, Chlor oder einer Mischung von Sauerstoff und Stickstoff ausgewähltes Oxidationsmittel enthaltenden Gasstrom umfasst.

12. Verfahren nach dem vorhergehenden Anspruch, wobei der Aktivierungsschritt einen auf den ersten Schritt folgenden zweiten Schritt des Behandelns des nach dem ersten Schritt erhaltenen Fluorierungskatalysators mit einem Gasgemisch, das ein Reduktionsmittel und ein Inertgas umfasst, umfasst, wobei es sich bei dem Reduktionsmittel um Wasserstoff oder einen C₂-C₆-Halogenkohlenwasserstoff handelt.

13. Verfahren nach einem der vorhergehenden Ansprüche, wobei die Schritte (a) und (b) alternierend durchgeführt werden.

14. Verfahren nach einem der vorhergehenden Ansprüche, wobei vor und/oder nach Schritt (b) eine Spülung des Reaktors durchgeführt wird, wobei die Spülung vorzugsweise durch Einleiten eines Stickstoffstroms in den Reaktor oder durch Halten des Reaktors unter Vakuum durchgeführt wird.

15. Verfahren zur Fluorierung eines 2,3,3,3-Tetrafluorpropens zu 1,1,1,2,2-Pentafluorpropan, das die folgenden Schritte umfasst:
(a) Inkontaktbringen von 2,3,3,3-Tetrafluorpropen mit Fluorwasserstoff in der Gasphase in Gegenwart eines Fluorierungskatalysators in einem Reaktor zur Herstellung des 1,1,1,2,2-Pentafluorpropans und
(b) Regenerieren des in Schritt a) verwendeten Fluorierungskatalysators,
wobei der Schritt (b) des Regenerierens des Fluorierungskatalysators (c) das Behandeln des Fluorierungskatalysators mit einem ein Oxidationsmittel enthaltenden Gasstrom zur Bildung eines oxidierten Fluorierungskatalysators und (d) das Behandeln des in Schritt (c) erhaltenen oxidierten Fluorierungskatalysators mit einer Gasmischung, die ein Reduktionsmittel und ein Inertgas umfasst, umfasst, wobei der in Schritt b) regenerierte Katalysator in Schritt a) wiederverwendet wird, wobei das Reduktionsmittel aus der Gruppe bestehend aus C₁-C₁₀-Halogenkohlenwasserstoffen ausgewählt wird und wobei der Katalysator aus der Gruppe bestehend aus Chromoxidfluorid, Chromoxiden, Chromhalogeniden und Mischungen davon ausgewählt wird.

## Claims

1. Process for fluorinating a chlorinated C₃ alkane or alkene compound containing at least one chlorine atom to a fluorinated C₃ alkane or alkene compound containing at least one fluorine atom, comprising the following steps:
(a) contacting, in a reactor, the chlorinated compound with hydrogen fluoride in gas phase in the presence of a fluorination catalyst to produce a fluorinated compound, the number of fluorine atoms in the fluorinated compound being greater than the number of fluorine atoms in the chlorinated compound, and
(b) regenerating the fluorination catalyst used in step a),
where the step (b) of regenerating the fluorination catalyst comprises (c) treating said fluorination catalyst with a gas flow containing an oxidizing agent to form an oxidized fluorination catalyst, and (d) treating the oxidized fluorination catalyst obtained in step (c) with a gaseous mixture comprising a reducing agent and an inert gas, the catalyst regenerated in step b) is reused in step a), the reducing agent is selected from the group consisting of C₁-C₁₀ hydrohalocarbons, and said catalyst is selected from the group consisting of chromium oxyfluoride, chromium oxides, chromium halides and mixtures thereof.

2. Process according to any of the preceding claims, wherein the inert gas is selected from nitrogen, helium, argon and mixtures thereof.

3. Process according to the preceding claim, **characterized in that**, when the reducing agent is a C₁-C₁₀ hydrohalocarbon, the gaseous mixture comprises HF.

4. Process according to any of the preceding claims, wherein the gaseous mixture of step (d) comprises from 1 to 10% by volume of reducing agent, preferably from 2 to 9% by volume, more preferably from 3 to 7% by volume, based on the total volume of the gaseous mixture.

5. Process according to any of the preceding claims, **characterized in that** the gaseous mixture from step (d) comprises a C₂-C₆ hydrohalocarbon, nitrogen or argon, and HF; preferably the gaseous mixture from step (d) comprises a C3 hydrohalocarbon, nitrogen and HF.

6. Process according to any of the preceding claims, wherein step d) is carried out at a temperature ranging from 100°C to 450°C, with a contact time of from 1 to 100 s, preferably of from 1 to 75 s, more preferably of from 5 to 50 s, for a time greater than 1 hour, preferably from 1 to 50 hours, in particular from 4 to 25 hours.

7. Process according to any of the preceding claims, wherein the chlorinated compound is 2-chloro-3,3,3-trifluoro-1-propene (HFCO-1233xf), 1,1,1,2,3-pentachloropropane (HCC-240db), 1,1,2,2,3-pentachloropropane (HCC-240aa), 2,3-dichloro-1,1,1-trifluoropropane (HCFC-243db), 1,1,2,3-tetrachloro-1-propene (HCO-1230xa), 2,3,3,3-tetrachloro-1-propene (HCO-1230xf), 2-chloro-1,1,1,2-tetrafluoropropane (HCFC-244bb), 1,1,1,3,3-pentachloropropane (HCC-240fa), 1,1,3,3-tetrachloropropene (HCO-1230za), 1,3,3,3-tetrachloropropene (HCO-1230zd), 1-chloro-3,3,3-trifluoropropene (HCFO-1233zd), 1,1,1,3-tetrachloropropane (HCC-250fb), 1,1,3-trichloropropene (HCO-1240za), 3,3,3-trichloropropene (HCO-1240zf); more preferably the chlorinated compound is selected from the group consisting of 2-chloro-3,3,3-trifluoro-1-propene (HFCO-1233xf), 1,1,1,2,3-pentachloropropane (HCC-240db), 1,1,2,2,3-pentachloropropane (HCC-240aa), 2,3-dichloro-1,1,1-trifluoropropane (HCFC-243db), 1,1,2,3-tetrachloro-1-propene (HCO-1230xa), 2,3,3,3-tetrachloro-1-propene (HCO-1230xf) or 2-chloro-1,1,1,2-tetrafluorocarbons (HCFC-244bb).

8. Process according to any of the preceding claims, wherein the fluorinated compound is 2,3,3,3-tetrafluoro-1-propene (HFO-1234yf), 2-chloro-1,1,1,2-tetrafluoropropane (HCFC-244bb), 1,1,1,2,2-pentafluoropropane (HFC-245cb), 1,1,1,3,3-pentafluoropropane (HFC-245fa), 1,1,1,3-tetrafluoro-3-chloropropane (HCFC-244fa), 1-chloro-3,3,3-trifluoropropene (HCFO-1233zd), 1,3,3,3-tetrafluoropropene (HFO-1234ze), 3,3,3-trifluoropropene (HFO-1243zf) or 2-chloro-3,3,3-trifluoro-1-propene (HFCO-1233xf) .

9. Process according to any of the preceding claims, wherein the fluorination of the chlorinated compound to a fluorinated compound is:
- 2-chloro-3,3,3-trifluoro-1-propene (HFCO-1233xf) to 2,3,3,3-tetrafluoro-1-propene (HFO-1234yf);
- 1,1,1,2,3-pentachloropropane (HCC-240db) to 2,3,3,3-tetrafluoro-1-propene (HFO-1234yf);
- 1,1,2,2,3-pentachloropropane (HCC-240aa) to 2,3,3,3-tetrafluoro-1-propene (HFO-1234yf);
- 2,3-dichloro-1,1,1-trifluoropropane (HCFC-243db) to 2,3,3,3-tetrafluoro-1-propene (HFO-1234yf);
- 1,1,2,3-tetrachloro-1-propene (HCO-1230xa) to 2,3,3,3-tetrafluoro-1-propene (HFO-1234yf);
- 2,3,3,3-tetrachloro-1-propene (HCO-1230xf) to 2,3,3,3-tetrafluoro-1-propene (HFO-1234yf);
- 1,1,1,2,3-pentachloropropane (HCC-240db) to 2-chloro-3,3,3-trifluoro-1-propene (HFCO-1233xf);
- 1,1,2,2,3-pentachloropropane (HCC-240aa) to 2-chloro-3,3,3-trifluoro-1-propene (HFCO-1233xf);
- 1,1,2,3-tetrachloro-1-propene (HCO-1230xa) to 2-chloro-3,3,3-trifluoro-1-propene (HFCO-1233xf);
- 2,3,3,3-tetrachloro-1-propene (HCO-1230xf) to 2-chloro-3,3,3-trifluoro-1-propene (HFCO-1233xf);
- 2-chloro-3,3,3-trifluoro-1-propene (HFCO-1233xf) to 2-chloro-1,1,1,2-tetrafluoropropane (HCFC-244bb);
- 1,1,1,2,3-pentachloropropane (HCC-240db) to 2-chloro-1,1,1,2-tetrafluoropropane (HCFC-244bb);
- 1,1,2,2,3-pentachloropropane (HCC-240aa) to 2-chloro-1,1,1,2-tetrafluoropropane (HCFC-244bb);
- 2,3-dichloro-1,1,1-trifluoropropane (HCFC-243db) to 2-chloro-1,1,1,2-tetrafluoropropane (HCFC-244bb);
- 1,1,2,3-tetrachloro-1-propene (HCO-1230xa) to 2-chloro-1,1,1,2-tetrafluoropropane (HCFC-244bb);
- 2,3,3,3-tetrachloro-1-propene (HCO-1230xf) to 2-chloro-1,1,1,2-tetrafluoropropane (HCFC-244bb);
- 1,1,1,2,3-pentachloropropane (HCC-240db) to 1,1,1,2,2-pentafluoropropane (HFC-245cb);
- 1,1,2,2,3-pentachloropropane (HCC-240aa) to 1,1,1,2,2-pentafluoropropane (HFC-245cb);
- 2,3-dichloro-1,1,1-trifluoropropane (HCFC-243db) to 1,1,1,2,2-pentafluoropropane (HFC-245cb);
- 1,1,2,3-tetrachloro-1-propene (HCO-1230xa) to 1,1,1,2,2-pentafluoropropane (HFC-245cb);
- 2,3,3,3-tetrachloro-1-propene (HCO-1230xf) to 1,1,1,2,2-pentafluoropropane (HFC-245cb).

10. Process according to the preceding claims, wherein the fluorination catalyst contains one or more cocatalysts comprising a salt of a transition metal selected from the group consisting of Co, Zn, Mn, Mg, V, Mo, Te, Nb, Sb, Ta, Ni; or a phosphorous salt.

11. Process according to any of the preceding claims, comprising an activation step carried out before step (a) which comprises a first step of contacting the fluorination catalyst with a gas flow containing an oxidizing agent selected from oxygen, air, chlorine or a mixture of oxygen and nitrogen.

12. Process according to the preceding claim, wherein the activation step comprises a second step, subsequent to the first step, of treating the fluorination catalyst obtained after the first step with a gaseous mixture comprising a reducing agent and an inert gas, said reducing agent being hydrogen or a C₂-C₆ hydrohalocarbon.

13. Process according to any of the preceding claims, wherein steps (a) and (b) are carried out alternately.

14. Process according to any of the preceding claims, wherein a purge of the reactor is carried out before and/or after step (b), the purge preferably being carried out by introducing a flow of nitrogen into the reactor or by maintaining the reactor under vacuum.

15. Process for fluorinating a 2,3,3,3-tetrafluoropropene to 1,1,1,2,2-pentafluoropropane, comprising the following steps:
(c) contacting, in a reactor, 2,3,3,3-tetrafluoropropene with hydrogen fluoride in gas phase in the presence of a fluorination catalyst to produce 1,1,1,2,2-pentafluoropropane, and
(d) regenerating the fluorination catalyst used in step a),
where the step (b) of regenerating the fluorination catalyst comprises (c) treating said fluorination catalyst with a gas flow containing an oxidizing agent to form an oxidized fluorination catalyst, and (d) treating the oxidized fluorination catalyst obtained in step (c) with a gaseous mixture comprising a reducing agent, the catalyst regenerated in step b) is reused in step a), the reducing agent is selected from the group consisting of C₁-C₁₀ hydrohalocarbons, and said catalyst is selected from the group consisting of chromium oxyfluoride, chromium oxides, chromium halides and mixtures thereof.
